# EUROPEAN PATENT APPLICATION

(11) **EP 1 635 553 A2**
(43) Date of publication of application: **15.03.2006**
(21) Application number: 05027319.2
(22) Date of filing: 05.02.2004
(51) Int. Cl.: H04N 1/32, H04N 1/21

(54) **Medical image processing system, medical image pickup system and method of administering medical images**

(30) Priority: 10.02.2003 JP 2003032127; 10.02.2003 JP 2003032350; 17.03.2003 JP 2003071377
(62) Divisional of application: 04250613.9
(71) Applicant: KONICA MINOLTA HOLDINGS, INC., Tokyo 100-0005 (JP)
(72) Inventor: Ishimitsu, Yoshiyuki, 2970 Ishikawa-machi Hachioji-shi Tokyo (JP); Umeki, Mamoru, 2970 Ishikawa-machi Hachioji-shi Tokyo (JP); Moriyama, Naoto, 2970 Ishikawa-machi Hachioji-shi Tokyo (JP); Shiibashi, Takao, 2970 Ishikawa-machi Hachioji-shi Tokyo (JP)
(74) Representative: Rees, Alexander Ellison

(57) **Abstract**

A medical image processing system having: an input section (10) for inputting image information of a patient; an output section (90) for visualizing the image information of a patient inputted from the input section; and an image-attached information recording section (70); wherein, the image-attached information recording section (70) records, as information attached to image information, at least one of information of the input section (10) which has been used for inputting the image information, and information of the cassette which has been loaded in the input section and used for inputting the image information or on the stimulable phosphor sheet loaded in the cassette.

## Description

### BACKGROUND OF THE INVENTION

This invention relates to a medical image processing system, medical image pickup system and a method of administrating medical images.

In recent years, it is actualized in hospitals that a plurality of input apparatus such as an image pickup apparatus such as a CT (Computed Tomography) apparatus and an MRI (Magnetic Resonance Imaging) apparatus and reading apparatus such as a CR (Computed Radiography) apparatus and an FPD (Flat panel Display) apparatus are connected with a network, while output apparatus such as an imager and a viewer are also connected with a network. Further, it is put into practice that image information of a patient inputted by means of an input apparatus that is most convenient in terms of operation flow (for example, an input apparatus located near a person who is in charge of the input such as a radiographer who inputs image information of a patient) is outputted by means of an output apparatus that is most convenient in terms of operation flow (for example, an output apparatus located near a doctor who carries out diagnosis of a patient by using said image information), and the overall efficiency of diagnosis is made higher (refer to the patent literature 1, for example).

Further, in cases where a plurality of doctors diagnose one and the same patient, sometimes image information of the patient is distributed to a plurality of output apparatus.

### [Patent literature 1]

The publication of the unexamined patent application 2002-159476.

Further, a radiation image pickup apparatus is one that uses a medical image conversion panel having a stimulable phosphor layer formed on a substrate, and a latent image is formed by the following process: that is, radiation transmitted through a radiographic object is absorbed by the stimulable phosphor layer of this conversion panel, in which radiation energy corresponding to the radiation transmittance of various parts of the radiographic object is accumulated. After that, by excitation light rays such as infrared rays scanning this phosphor layer, the accumulated radiation energy is made to emit fluorescent light, which is converted into electricity, to give a medical image signal. A medical image obtained in this way, having been subjected to image processing, is outputted to a film or an output device such as a CRT to become visualized, or preserved in a filing device such as a server together with some bits of information of the patient, to be utilized in medical activity.

As regards a medical image radiographing system utilizing an image pickup apparatus as described above, the system structure employed is generally classified into two. One is a conventional medical image system in which an apparatus for picking up and reading an image and a stimulable phosphor plate are arranged in or in the neighborhood of an image pickup room, and the image pickup is carried out in the image pickup room. In this system, it is possible to carry out the reading of an image at the same time of the pickup of the image in the image pickup room. ,

The other is a medical image system in which radiographing is carried out in the field of the round of visits of a doctor, for patients who cannot be subjected to radiographing in an image pickup room such as a patient of a broken bone or of a disease of cerebral veins or patients under control in a centralized treatment room, by the utilization of a movable image pickup apparatus for round of visits and a portable cassette with a built-in stimulable phosphor plate (refer to the patent literature 2, for example). In this system, the reading of an image is carried out through the insertion of the cassette into a reading device dedicated to a cassette after the image pickup.

In such a medical image pickup system, as described above, a plurality of apparatus such as an image pickup apparatus and image reading apparatus installed in an image pickup room, a movable image pickup apparatus, and a reading apparatus dedicated to a cassette are utilized. Therefore, in order that a medical image of the same image quality can be provided, even in the case where different image pickup apparatus and reading apparatus are utilized, information which is necessary for image processing is added to the radiographed image data as attached bits of information (refer to the patent literature 3, for example). In another way, it is known a medical information administration apparatus capable of editing the attached bits of information to be added to image data in order to make such a system be capable of extension (refer to the patent literature 4, for example).

### [Patent literature 2]

The publication of the unexamined patent application 2000-139885.

### [Patent literature 3]

The publication of the unexamined patent application S62-150474.

### [Patent literature 4]

The publication of the unexamined patent application 2002-133394.

### (PROBLEM TO BE SOLVED BY THE INVENTION)

Now, as regards input apparatus, it has been actualized that what is called a decentralized processing system in which a plurality of cassettes (plates) are made to be used in a plurality of reading apparatus in a computed radiography apparatus for example, and an FPD integrally made of both cassette and reading apparatus also has come to be used. In this way, the route for supplying a diagnosis image to an output apparatus, the combination of apparatus to be used, etc. have become complex.

Further, it cannot be said that the same image information is obtained by all of these various kinds of apparatus used, but for example, the image information is subject to the influence of the deterioration of the function of the apparatus accompanied by its being used and to the influence of the environment (dusts etc.).

In this way, at the sending-out side of diagnosis information (input apparatus), in cases where some failure occurs in diagnosis information accompanied by the decentralized arrangement of the apparatus with the work flow in the hospital taken into consideration, the specification of the cause has come to be a difficult operation requiring a considerably long time.

On the other hand, as regards the output apparatus, for example, there is an imager of a type in which a full-frame image on a film can be obtained by a main scanning using a polygonal mirror and a sub-scanning caused by film conveyance; however, sometimes there is a difference in the performance between one and another of the apparatus, and for example, sometimes the finished images do not become quite the same even if the same data are visualized by the imagers due to the difference of the beam diameter in the main scanning or the unevenness of feed in the sub-scanning.

That is, if the number of imager used is one, the same images are easy to obtain from the same data, which gives comparatively a little influence to diagnosis; however, if a plurality of imagers are used, due to the difference of the performance among the apparatus, there is a possibility of images being in different states even from the same data, which gives some apprehension about the influence to diagnosis. In particular, in such a case that the states of cure of a focus of the same patient changing with the passage of time are compared, and in the case where an image radiographed this time is compared with the image of one month before and with the image of two months before, if the image is outputted by means of a different imager from the others this time only, it sometimes occurs that the state of the image become different from the others (for example, the state of image becomes inferior to the other images) owing to the difference in the performance between the imagers, and it can be considered that the diagnosis by the image become erroneous because of this point.

Further, also in cases where a failure is generated in the imager owing to an insufficient maintenance of the imager, no version upgrading having been carried out for the control software of the imager, or the like, sometimes the state of the image become different from the others.

In such cases, on the basis of the observation such that the patient is properly to be getting better, the output to a film is done over again on the basis of existing data, or the output is done over again from the image input, and the pursuit of the cause is to be carried out; this has been an operation requiring a considerable amount of time and number of working hours, and exhausting large amount of resources.

Besides, in cases where a plurality of imagers are arranged in accordance with the work flow in the hospital in the same way as the case of the input apparatus, or in the case where these imagers make backup mutually to become complementary to one another, the pursuit of the cause of failures in the case where an extensive view of the whole from the input to the output is taken becomes further more difficult.

Incidentally, because a plurality of apparatus are utilized in a conventional medical image pickup system as described in the above, in cases where an abnormality is generated in a radiographed medical image, it has been difficult to identify in which process the abnormality is generated among a sequence of radiographing processes. That is, in a conventional medical image pickup system, supplementary information attached to image data has been information to be added for the purpose of forming a high-quality image, and it has not been the information to be utilized for the pursuit of cause of an abnormality being generated.

Further, the cause of an abnormality being generated is not only a failure in the apparatus such as the image pickup apparatus and the reading apparatus, but also in the case where a medical image pickup system for carrying out the radiographing at a site of round of visits, sometimes the radiographing environment at the site of round of visits, the cassette used, etc. In another case, sometimes the cause of an abnormality being generated is the technical level of the radiographer carrying out the image pickup.

It is an object of this invention to provide a medical image pickup system and a method of controlling medical images which make it possible, in cases where an abnormality is generated in a medical image, to identify the cause of the abnormality being produced easily and speedily, to remove the abnormality quickly.

### SUMMARY OF THE INVENTION

The above-mentioned problems can be solved by an invention having any one of the features described below.
(1) A medical image processing system equipped with
   an input section for inputting image information of a patient, and
   an output section for visualizing image information of a patient inputted from said input section, characterized by being further equipped with
   an input information recording section (image-attached information recording section) capable of recording, as information attached to image information, at least one of
   input section information on the input section which has been used in the inputting of image information, and
   information of the cassette which has been loaded in said input section and used in the inputting of image information or on the stimulable phosphor sheet loaded in the cassette.
   According to the invention having the feature described in (1), at least one of input section information enabling the identification of the input section (reader) used in the inputting, and cassette information enabling the identification of the cassette loaded in said input section and used in the inputting of image information or the stimulable phosphor sheet accommodated in the cassette is made to be recorded; therefore, in cases where a failure of image information is generated, it can be immediately judged whether or not the cause is in the input section and/or in the cassette by the mere confirmation of the input section and/or the cassette concerned.
(2) A medical image processing system having a feature as set forth in the feature (1), characterized by
   being further equipped with an image information confirming section for displaying to confirm image information inputted from the aforesaid input section, and
   being capable of recording information indicating the existence of a defect as information attached to image information, in cases where a defect of image information is discovered at the time of confirmation of image information in said image information confirming section.
   According to the feature (2), the medical image processing system is equipped with an image information confirming section for displaying to confirm image information such as a viewer, and is capable of recording information indicating the existence of a defect as information attached to image information, in cases where the existence of a defect in image information is discovered by the display for confirmation (for example, disturbance in image information due to dusts etc.); therefore, discrimination between normal image information and defective image information can be easily made, and after that, the pursuit of the cause of the failure can be quickly carried out.
(3) A medical image processing system having a feature as set forth in the feature (2), characterized by being further equipped with
   an image saving section for storing and saving image information and image-attached information inputted,
   a defect information detecting section for detecting information indicating the existence of a defect from the image-attached information stored and saved in said image saving section, and
   an identification section for detecting and analyzing at least one of input section information and cassette information out of the image-attached information from which information indicating the existence of a defect has been detected and identifying at least one of said input section and cassette or stimulable phosphor sheet.
   According to the feature (3), in cases where image information has a defect, at least one of the aforesaid input section and cassette or stimulable phosphor sheet which have been used in the inputting of image information having a defect is automatically identified; therefore, the pursuit of the cause of the failure can be carried out more simply.
(4) A medical image processing system having a feature as set forth in any one of the features (1) to (3), characterized by the aforesaid image information being transmitted and received between the aforesaid sections by means of a standard signal based on DICOM standard.
   In the above, DICOM (Digital Imaging and Communications in Medicine) standard is an industrial standard protocol specifying the transmission of an image and other kinds of medical information between computers, and it enables digital communication between diagnosis apparatus or treatment apparatus of different makers.
   According to the feature (4), image information is transmitted and received between the sections concerned by means of a standard signal based on DICOM standard; therefore, it is possible to cope with various kinds of input apparatus and output apparatus.
(5) A medical image processing system equipped with
   an input section for inputting image information of a patient, and
   an output section for outputting image information of a patient inputted from said input section to a film, characterized by
   being further equipped with an input/output section information recording section capable of recording on a photosensitive film, at least one of information of the input section used in the inputting of image information, output section information on the output section used in the outputting of image information, and film information on the photosensitive film used in the outputting of image information.
   According to the feature (5), it is actualized that, when image information is outputted to a photosensitive film in an output section such as an imager, at least one of input section information enabling the identification of the reader used in the inputting, output section information enabling the identification of the imager having carried out the outputting concerned, and film information on the photosensitive film used at the time of carrying out the outputting concerned is recorded; therefore, in cases where a failure of image information is generated, it is possible to immediately judge whether or not the cause is in the input section, output section, and/or film by mere confirmation of the input section, output section, and film concerned, and the pursuit of the cause of the failure can be easily carried out.
(6) A medical image processing system equipped with
   an input section for inputting image information of a patient, and
   an output section for visualizing image information of a patient inputted from said input section, characterized by being further equipped with
   an input/output section information correlating section for correlating information of the input section used in the inputting of image information and output section information on the output section used in the visualizing of image information, and
   an input/output section information recording section capable of recording input section information and output section information correlated by said input/output section information correlating section.
   According to the feature (6), it is actualized that, in the case where image information is outputted for display in the output section such as a viewer, input section information enabling the identification of the reader used in the inputting and output section information enabling the identification of the viewer used in the outputting concerned are correlated and then recorded; therefore, in cases where a failure of image information is generated, it is possible to immediately judge whether or not the cause is in the input section and/or output section by mere confirmation of the input section and output section concerned, and the pursuit of the cause of the failure can be easily carried out.
(7) A medical image processing system having a feature as set forth in the feature (6), characterized by
   the aforesaid input/output section information correlating section being capable of correlating also the result information of diagnosis carried out on the basis of the aforesaid visualized image information of a patient, and
   the aforesaid input/output section information recording section being capable of recording input section information, output section information, and diagnosis result information correlated mutually by said input/output section information correlating section in relation to the image information.
   According to the feature (7), it is actualized that, in the case where image information is outputted for display in an output section such as a viewer and diagnosis is carried out on the basis of said image information, diagnosis result information, together with input section information and output section information, is also recorded in relation to the image information; therefore, in cases where a failure of image information is generated, it is possible to immediately judge where the cause is by the mere confirmation of the specified sections, and the pursuit of the cause of the failure can be easily carried out.
(8) A medical image processing system equipped with
   an input section for inputting image information of a patient, and
   an output section for visualizing image information of a patient inputted from said input section, characterized by being further equipped with
   an input section information recording section capable of recording information of the input section used in the inputting of image information as information attached to image information, and
   an output section recording section capable of recording output section information on the output section used in the visualizing of said image information in addition to said attached bit of information having said input section information recorded.
   According to the feature (8), it is actualized that, when image information is inputted, input section information enabling the identification of the reader used in the inputting concerned is recorded as an attached bit of information, and when image information is outputted for display in an output section such as a viewer, output section information enabling the identification of the viewer used in the outputting concerned is further recorded as an attached bit of information; therefore, in cases where a failure of image information is generated, it is possible to immediately judge whether or not the cause is in the input section and/or output section by mere confirmation of the input section and output section concerned, and the pursuit of the cause of the failure can be easily carried out.
(9) A medical image processing system having a feature as set forth in the feature (8), characterized by being further equipped with diagnosis result information recording section capable of recording the result information of diagnosis carried out on the basis of the aforesaid visualized image information of a patient in addition to the attached bit of information having the aforesaid input section information recorded, and
   the aforesaid output section information recording section being capable of recording output section information on the output section used in the visualizing of the image information concerned in addition to said attached bit of information having said input section information and diagnosis result information recorded.
   According to the feature (9), it is actualized that, when image information is inputted, input section information enabling the identification of the reader used in the inputting concerned is recorded as an attached bit of information, and when image information is outputted for display in an output section such as a viewer, output section information enabling the identification of the viewer used in the outputting concerned is recorded as an attached bit of information, while result information of the diagnosis carried out on the basis of image information is recorded as an attached bit of information; therefore, in cases where a failure of image information is generated, it is possible to immediately judge where the cause is by the mere confirmation of the specified sections, and the pursuit of the cause of the failure can be easily carried out.
(10) A medical image processing system having a feature as set forth in the feature (5), characterized by the aforesaid input/output section information recording section being capable of recording status information of the input section and/or status information of the output section on a photosensitive film.
   In the above description, for the status information, for example, information on the maintenance of an apparatus, version upgrading information of a control software for controlling an apparatus can be cited.
   According to the feature (10), it is actualized that status information of an input section and that of an output section are recorded in relation to input section information and output section information; therefore, in cases where a failure of image information is generated, it is possible to immediately judge where the cause is more simply, and the pursuit of the cause of the failure can be easily carried out.
(11) A medical image processing system having a feature as set forth in the feature (6) or (7), characterized by the aforesaid input/output section information recording section being capable of recording status information of the input section and/or status information of the output section in relation to image information.
   According to the feature (11), it is actualized that status information of the input section and that of the output section are recorded in relation to the input section information and the output section information; therefore, in cases where a failure of image information is generated, it is possible to immediately judge where the cause is more simply, and the pursuit of the cause of the failure can be easily carried out.
(12) A medical image processing system having a feature as set forth in the feature (8) or (9), characterized by
   the aforesaid input section information recording section being capable of recording status information of the input section as information attached to image information, and
   the aforesaid output section information recording section being capable of recording status information of the output section as information attached to image information.
   According to the feature (12), it is actualized that status information of the input section and that of the output section are recorded in relation to the input section information and the output section information; therefore, in cases where a failure of image information is generated, it is possible to immediately judge where the cause is more simply, and the pursuit of the cause of the failure can be easily carried out.
(13) A medical image pickup system equipped with a control apparatus for controlling image pickup order information and a medical image in correspondence with each other, and a portable terminal for acquiring image pickup order information from said control apparatus, characterized by
   said portable terminal being equipped with
   an acquisition means for acquiring at least one or more of identification information of a cassette having a medical image recorded, identification information of a radiographer having carried out the image pickup, and identification information of an image pickup apparatus having recorded a medical image in a cassette,
   a storage means for storing the above-mentioned acquired bits of identification information in correspondence with the image pickup order information concerned, and
   a communication device for transmitting said bits of identification information in correspondence with the image pickup order information concerned, and
   said control apparatus being equipped with
   a communication device for receiving said bits of identification information brought in correspondence with the image pickup order information concerned, and
   an image pickup history administration file for storing said received bits of identification information in correspondence with the image pickup order information concerned.
   Accordingly, in correspondence with image pickup order information, identification information of a radiographer having carried out the image pickup process, identification information of a cassette having a medical image recorded, and identification information of an image pickup apparatus having recorded a medical image are stored; therefore, it is possible to identify the radiographer, the cassette, and the image pickup apparatus having been involved in the image pickup process speedily.
(14) A medical image pickup system having a feature as set forth in the feature (13), characterized by being further equipped with
   a reading apparatus for reading, from a cassette having a medical image recorded, a medical image and the identification information of the cassette,
   said reading apparatus being equipped with a communication device for transmitting identification information of the cassette and identification information of the reading apparatus having carried out the reading of said medical image in correspondence with said medical image,
   in the aforesaid control apparatus,
   the aforesaid communication device receiving the medical image brought in correspondence with said identification information of the cassette and said identification information of the reading apparatus, and
   the aforesaid image pickup history administration file storing said identification information of the reading apparatus in correspondence with the bits of identification information including said identification information of the cassette, and
   said medical image pickup system being further equipped with a control device (an administration device) for controlling said medical image and said image pickup order information in correspondence with each other on the basis of said identification information of the cassette.
   Accordingly, it is possible that the radiographer, cassette, the image pickup apparatus, and the reading apparatus involved in the image pickup process are stored in the image pickup history administration file and controlled in correspondence with the image pickup order information, while the medical image is controlled in correspondence with the image pickup order information. Owing to this, for example, in cases where an abnormality is generated in a medical image, on the basis of the image pickup history administration file, the cause of the abnormality can be searched through the inspection of various factors to becomes the cause such as the radiographer, cassette, image pickup apparatus, reading apparatus, etc. involved in the image pickup process, and the cause of the generation of abnormality can be canceled with certainty.
(15) A medical image pickup system having a feature as set forth in the feature (14), characterized by
   the aforesaid control device (administration device) making the aforesaid medical image have at least one or more of the aforesaid identification information of the cassette, identification information of the radiographer, identification information of the image pickup apparatus, and identification information of the reading apparatus attached as attached bits of information.
   Accordingly, it is possible that a medical image is made to have at least one or more of the identification information of a cassette, the identification information of a radiographer, the identification information of an image pickup apparatus, and the identification information of a reading apparatus attached as attached bits of information and these are controlled in correspondence with the image pickup order information; therefore, the correspondence relation between a medical image and various kinds of identification information can be made definite, and the loss of information can be prevented.
(16) A medical image pickup system having a feature as set forth in the feature (13) or (15), characterized by
   the aforesaid control apparatus being equipped with
   a display control device for making a display section display the aforesaid medical image, and
   an input control device for inputting error information indicating whether or not an abnormality is present in said medical image displayed, and
   the aforesaid image pickup history administration file storing error information in correspondence with the above-mentioned bits of identification information corresponding to said medical image.
   Accordingly, by the storage of the occurrence of an abnormality for medical images having ever had an abnormality generated, the causes of abnormality can be extracted statistically on the basis of medical images having ever had an abnormality generated; therefore, the cause of abnormality generated in a medical image can be searched easily with a high efficiency.
(17) A method of administrating medical images in a medical image pickup system equipped with a control apparatus for controlling image pickup order information and a medical image in correspondence with each other, and a portable terminal for acquiring image pickup order information from said control apparatus, characterized by comprising
   a process of acquiring at least one or more of identification information of a cassette having a medical image recorded, identification information of a radiographer having carried out the image pickup, and identification information of an image pickup apparatus having recorded a medical image in a cassette,
   a process of storing the above-mentioned acquired bits of identification information in correspondence with the image pickup order information concerned,
   a process of transmitting said bits of identification information in correspondence with the image pickup order information concerned,
   a process of receiving said bits of identification information brought in correspondence with the image pickup order information concerned, and
   a process of storing said received bits of identification information in correspondence with the image pickup order information concerned.
(18) A method of administrating medical images having a feature as set forth in the feature (17), characterized by further comprising
   a process of reading, from a cassette having a medical image recorded, the medical image and the identification information of the cassette,
   a process of transmitting the identification information of the cassette and the identification information of the reading apparatus having carried out the reading of said medical image in correspondence with said medical image,
   a process of receiving the medical image having the identification information of the cassette and the identification information of the reading apparatus brought in correspondence,
   a process of storing said identification information of the reading apparatus in correspondence with various kinds of identification information including said identification information of the cassette, and
   a process of administrating said medical image and image pickup order information in correspondence with each other on the basis of said identification information of the cassette.
(19) A method of administrating medical images having a feature as set forth in the feature (18), characterized by further comprising a process of attaching one or more of the aforesaid identification information of the cassette, identification information of the radiographer, identification information of the image pickup apparatus, and identification information of the reading apparatus to the aforesaid medical image in correspondence with it.
(20) A method of administrating medical images having a feature as set forth in any one of the features (17) to (19), characterized by further comprising
   a process of displaying the aforesaid medical image on a display section,
   a process of inputting error information indicating whether or not an abnormality is present in said medical image displayed, and
   a process of storing error information in correspondence with the above-mentioned bits of information corresponding to said medical image.

### BRIEF DESCRIPTION OF THE DRAWINGS

FIG. 1 is a drawing showing the system structure of a medical image processing system of the embodiment of this invention;
FIG. 2 is a drawing showing the functional structure of the medical image generating apparatus shown in FIG. 1;
FIG. 3 is a drawing showing the functional structure of the candidacy abnormal shadow detecting apparatus shown in FIG. 1;
FIG. 4 is a drawing showing the system structure of a medical image pickup system 1100 in the embodiment of this invention;
FIG. 5 is a block diagram showing the structure of the essential part of the portable terminal 110 shown in FIG. 4;
FIG. 6 is a drawing showing the data structure of an image pickup order information file 1161 stored in a storage device 16 of FIG. 5;
FIG. 7 is a block diagram showing the structure of the essential part of the control apparatus 120 shown in FIG. 4;
FIG. 8 is a drawing showing the data structure of an image pickup history administration file to be stored in a memory apparatus 126 shown in FIG. 7;
FIG. 9(a) is a flow chart showing an image pickup preparation processing to be practiced by a CPU 121 of the control apparatus 120;
FIG. 9(b) is a flow chart showing an image pickup preparation processing to be practiced by a CPU 111 of the portable terminal 110;
FIG. 10 is a drawing showing an example of a menu screen to be displayed on a display 123 of the control apparatus 120;
FIG. 11 is a drawing showing an example of a portable list reception screen to be displayed on the display 123 of the control apparatus 120;
FIG. 12(a) is a drawing showing an example of an input screen for inputting patient information to be displayed on the display 123 of the control apparatus 120;
FIG. 12(b) is a drawing showing an example of an input screen for inputting image pickup information to be displayed on the display 123 of the control apparatus 120;
FIG. 13 is a flow chart showing an image pickup start processing to be practiced by the CPU 111 of the portable terminal 110;
FIG. 14(a) is a drawing showing an example of patient list screen to be displayed on the display 113 of the portable terminal 110;
FIG. 14(b) is a drawing showing an example of display screen to be displayed on the display 113 of the portable terminal 110;
FIG. 15 is a flow chart showing a post-image-pickup processing to be practiced by the CPU 121 of the control apparatus 120;
FIG. 16 is a drawing showing an example of a portable processing screen to be displayed on the display 123 of the control apparatus 120; and
FIG. 17 is a drawing showing the system structure of a medical image pickup system of another mode of the embodiment of this invention.

### DETAILED DESCRIPTION OF THE PREFERRED EMBODIMENT

In the following, the embodiment of this invention will be explained in detail with reference to the drawings.

### (EBBODIMENT 1 OF THE INVENTION)

In this embodiment, it will be explained an example of medical image processing system having a structure such that image information of a patient having been inputted from one of a plurality of input sections is outputted by one of a plurality of output sections, wherein through which channel the image information of a patient is outputted is recorded.

First, the structure will be explained.

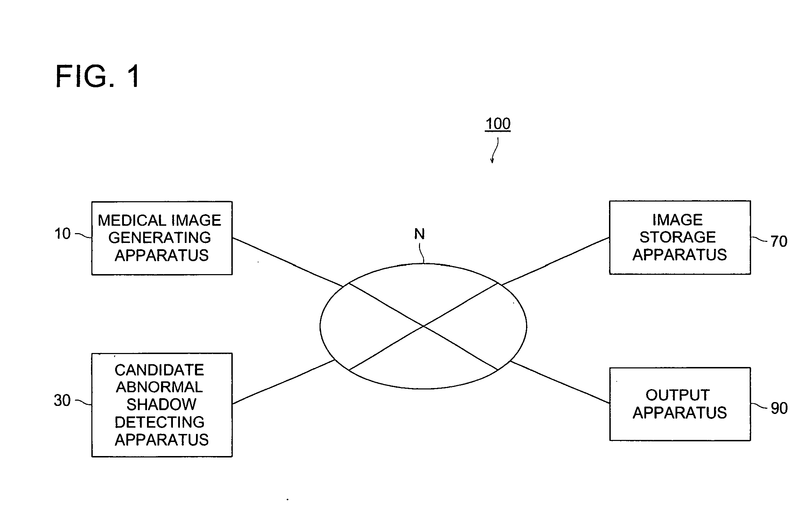

In FIG. 1, the system structure of a medical image processing system 100 of this embodiment is shown.

As shown in FIG. 1, the medical image processing system 100 is composed of a medical image generating apparatus 10 as an input section, a candidacy abnormal shadow detecting apparatus 30 as a diagnosis section, an image storage apparatus 70 as an image saving section, and an output apparatus 90 as an output section, and the apparatus are connected to one another through a communication network N in such a way as to be capable of transmitting and receiving information. Further, the candidacy abnormal shadow detecting apparatus 30, the image storage apparatus 70, and the output apparatus 90 are medical image receiving apparatus for receiving medical image data generated (image information of a patient) from the medical image generating apparatus 10.

Besides, although the apparatus are shown in FIG. 1 in a way such that one apparatus for each kind of them is connected to the network N; however, the place of installment and the number of apparatus are not limited to this example, but it is possible that a plurality of apparatus for each of the plural kinds of apparatus are installed. Further, also it is appropriate that other apparatus concerning the processing of medical images such as terminal apparatus installed in every medical treatment department are connected to the communication network N.

The communication network N is a LAN (Local Area Network) to which various kinds of communication lines such as a telephone line, a dedicated line, a mobile communication network, a communication satellite network, and a CATV line can be applied. Besides, also it is appropriate to apply various kinds of network type such as a WAN (Wide Area Network) and the Internet, but because medical images transmitted and received through the communication network N between the apparatus are personal information of patients, from the viewpoint of reliability in the information control, it is desirable that the communication network N is a dedicated network with its security kept certainly.

Further, in this description, image information of a patient (medical image data) and information such as an attached bit of information to be described later are supposed to be transmitted and received between the above-mentioned sections by means of a standard signal based on DICOM (Digital' Imaging and Communications in Medicine) standard. By this, digital communication between diagnosis apparatus or treatment apparatus of different makers is enabled.

In the following, every apparatus will be explained.

The medical image generating apparatus (input section) 10 generates digital image data (image information) for a radiographed medical image of a patient. In this embodiment, as regards the medical image generating apparatus, it will be explained an example to which a CR (Computed Radiography) apparatus which reads a radiographed radiation image of a patient and converts it into digital image data is applied.

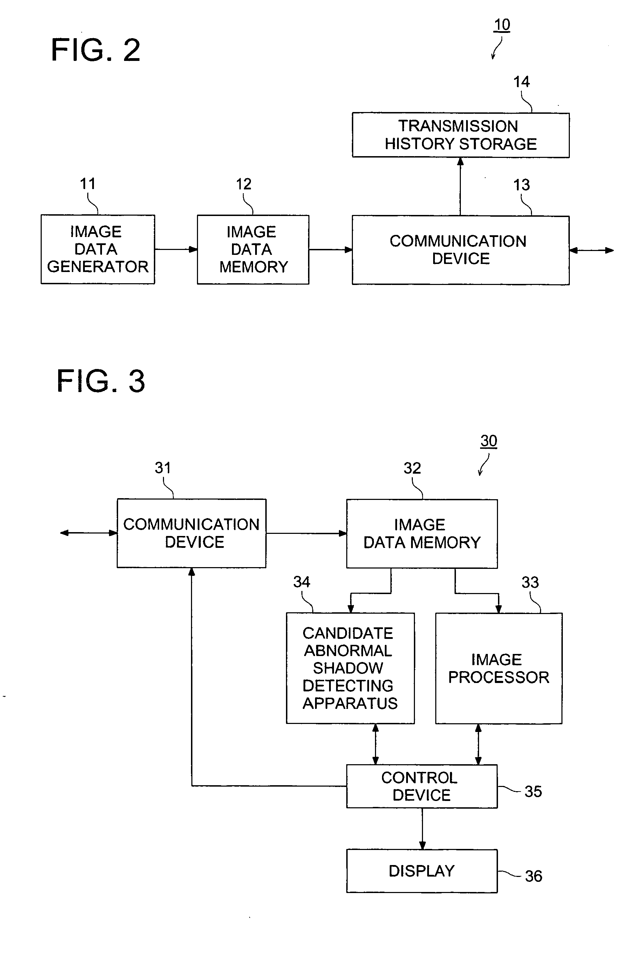

In FIG. 2, the functional structure of the medical image generating apparatus 10 is shown.

As shown in FIG. 2, the medical image generating apparatus 10 is equipped with an image data generator 11, an image data memory 12, a communication device 13, a transmission history storage 14, etc.

The image data generator 11 is composed of a cassette accommodating an image pickup panel (a stimulable phosphor sheet) having a stimulable phosphor layer formed, an excitation light generation device, a photoelectric conversion element such as a CCD (Charge Coupled Device) image sensor, an A/D converter, etc. The image data generator 11 makes radiation transmitted through an object be absorbed by an image pickup panel, and irradiates this image pickup panel with excitation light to scan the image pickup panel. Further, it photoelectrically converts the fluorescent light emitted from the image pickup panel into an analog image signal, and further generates digital image data through the A/D conversion of this analog image signal. Generated image data are outputted to the image data memory 12.

In addition, for the image data generator 11, a flat panel detector (Flat Panel Detector; hereinafter referred to as an FPD) is applicable. An FPD, as described in the publication of the unexamined patent application H6-342098 for example, has radiation detecting elements for generating electric charge corresponding to the intensity of radiation irradiating the elements and capacitors for accumulating the electric charge generated by this radiation detecting elements two-dimensionally arranged, and picks up a radiation image of an object to output it as an electrical signal.

Further, the image data generator 11 provides a header area for recording information on the medical image concerned in the medical image data generated (image information), and records, as bits of information attached to image, various kinds of patient information such as the name of the patient radiographed, the patient ID (ID for identifying the patient), and the name of the doctor in charge of the patient, and various kinds of image pickup information such as the image pickup condition at the time of image pickup (image pickup region, direction of image pickup), and the date and time of image pickup.

Further, in this embodiment, the image data generator 11 is equipped with a function as an input information recording section. Owing to this, it is actualized that, in the header area of said medical image data (image information), in addition to the above-mentioned various kinds of information, information on the input section used in the inputting of image information is recorded as input information, and information on the cassette (or the stimulable phosphor sheet accommodated in the cassette) loaded in said input section and used in the inputting of image information is recorded as cassette information.

In addition, for the input section information, for example, it can be cited an individual bit of information to be recorded for identifying the medical image generating apparatus (input section) having generated the image information concerned. For this individual bit of information, it can be cited the case where individual numbers are previously attached to their respective input sections (respective medical image generating apparatus) and the number concerned is recorded.

Further, for the cassette information, for example, it can be cited an individual bit of information to be recorded for identifying the cassette (or the stimulable phosphor sheet accommodated in the cassette) having generated the image information concerned. For this individual bit of information, it can be cited the case where individual numbers are previously attached to their respective cassettes (or respective stimulable phosphor sheets) and the number concerned is recorded.

Further, the image data generator 11 records, in the above-mentioned header area, information on the inspection to which the medical image belongs such as the inspection ID (ID for individually identifying the inspection) and the inspection requesting department. Because there is a case where one and the same patient is radiographed a plurality of times in different directions or by different image pickup apparatus in a single inspection, the same inspection ID is attached to image data belonging to one and the same inspection, and when the medical image data are transmitted, one or a plurality of sets of medical image data having the same inspection ID attached are collectively transmitted as a series of data group.

The image data memory 12 is made up of a magnetic or optical recording medium, a semiconductor memory, or the like, and stores medical image data (image information) generated by the image data generator 11.

The communication device 13 is an interface capable of being connected to the communication network N, and is composed of a network interface card, a modem, a terminal adapter, etc. The communication device 13 transmits medical image data (image information) stored in the image data memory 12 to the transmission end such as the candidacy abnormal shadow detecting apparatus 30 and the image storage apparatus 70.

The transmission history storage 14 is made up of a magnetic or optical recording medium, semiconductor memory, or the like, and saves information on the transmission history of medical image data transmitted through the communication device 13. For the information on the transmission history, for example, bits of information such as the name of image file of medical image data transmitted and the date and time of transmission are saved.

Further, in this embodiment, the medical image data generating means (input section) 10 is equipped with an image information confirming section (the drawing omitted) for displaying inputted image information for confirmation. Further, if a defect of image information (disturbance of the image, etc.) is discovered at the time of this confirmation, by a manual input means (the drawing omitted, included in the input information recording section), information indicating the existence of a defect can be recorded as information attached to image information.

In addition, the medical image generating apparatus is not limited to the above-mentioned CR apparatus, but it is also appropriate that, by the application of a laser digitizer, digital image data is generated by a process such that a film having a medical image recorded thereon is scanned by a laser beam, the transmitted light quantity is measured, and the light quantity value is subjected to digital conversion. It is also appropriate to generate medical image data by the application of a computerized sectional radiography apparatus (hereinafter referred to as a CT apparatus: a Computed Tomography apparatus), an MRI apparatus, an ultrasonic image reading apparatus, etc.

Next, the candidacy abnormal shadow detecting apparatus 30 will be explained.

The candidacy abnormal shadow detecting apparatus (diagnosis section) 30 is an image processing apparatus which detects an image area appearing to be an abnormal shadow and outputs it as a candidacy abnormal shadow by carrying out an image analysis of medical image data.

In FIG. 3, the functional structure of the candidacy abnormal shadow detecting apparatus 30 is shown.

As shown in FIG. 3, the candidacy abnormal shadow detecting apparatus 30 is equipped with a communication device 31, an image data memory 32, an image processor33, a candidacy abnormal shadow detecting device 34, a control device 35, and a display 36.

The communication device 31 is an interface capable of connection to the communication network N, and is composed of a network interface card, a modem, a terminal adapter, etc. The communication device 31 receives medical image data (image information of a patient) from the medical image generating apparatus 10 for example, and transmits medical image data (image information of a patient) having finished being subjected to a processing of the detection of a candidacy abnormal shadow and the result of the detection to the output apparatus 90.

The image data memory 32 is made up of a magnetic or optical recording medium, a semiconductor memory, or the like, and stores image data received by the communication device 31.

The image processor 33 applies various kinds of image processing to medical image data inputted from the image data memory 32, and outputs the data to the control device 35. The above-mentioned various kinds of image processing include a gradation processing for adjusting image contrast, a contrast correction processing for detecting a candidacy abnormal shadow, a frequency enhancement processing for adjusting the sharpness of an image, a dynamic range compression processing for making an image having a wide dynamic range fall within a density range easy to observe without lowering the contrast of minute portions of the radiographic object, etc.

The candidacy abnormal shadow detecting device 34 detects an image area appearing to be an abnormal shadow by reading out image data from the image data memory 32 and carrying out an image analysis. In this embodiment, an example in which a candidacy abnormal shadow is detected from a mammogram will be explained; however, the object of the detection is not limited to this, and it is also possible that some other region such as a chest or an abdomen is radiographed and a candidacy abnormal shadow in accordance with the region is detected from the image.

In mammography, a shadow seeming to be a phyma characterizing a breast cancer or a minute calcified cluster is detected. A shadow of a phyma is a lump having a size of a certain degree, and on a mammogram, it appears as a whitish round shadow having a density distribution near a Gaussian distribution. As regards a minute calcified cluster, if minute calcified portions exist in a congregation (making a cluster), the area has a high possibility to indicate a cancer in the early stage. On a mammogram, it appears as a whitish round shadow having approximately a conical shape.

Besides, it is put into practice to record the result of image analysis by means of the above-mentioned candidacy abnormal shadow detecting device 34 in relation to the medical image concerned by a diagnosis result information recording section (in this embodiment, this is included in the candidacy abnormal shadow detecting device 34). In this embodiment, it is recorded in such a manner as to be attached to the data of the image information.

Further, the candidacy abnormal shadow detecting device 34 in this embodiment is provided with a function as a diagnosis section information recording section which records the information on the diagnosis section used in the diagnosis of image information (the candidacy abnormal shadow detecting apparatus 30) in the header area of the above-mentioned medical image data (image information).

By this, in a case where there are plurality of the above-mentioned diagnosis sections (the candidacy abnormal shadow detecting apparatus 30) or in some case like that, it is possible to identify the diagnosis section (the candidacy abnormal shadow detecting apparatus 30) that has diagnosed the image information concerned.

To state it concretely, it can be cited a case where individual numbers are previously attached to their respective diagnosis sections (the candidacy abnormal shadow detecting apparatus 30), and the number concerned is recorded.

The control device 35 is composed of a CPU (Central Processing Unit), etc., and practices an output control of medical image data to be outputted to the communication device 31 or the display 36. The control device 35 outputs the information on the detection result by the candidacy abnormal shadow detecting device 34 to the communication device 31, together with the medical image data having been subjected to image processing by the image processor 33. Further, the control device 35, when outputting medical image data having been subjected to image processing to the 34, on the basis of the detection result by the candidacy abnormal shadow detecting device 34, outputs an image area of a candidacy abnormal shadow in such a way as to make it possible to identify the area of the candidacy abnormal shadow by the marking with an arrow mark, the changing of the color, or the like.

As regards the display means 36, a CRT (Cathode Ray Tube), an LCD, a plasma display, etc. can be employed for it, and among them, a high-definition, high-brightness CRT or liquid crystal display dedicated to medical images is desirable.

Next, the image storage apparatus 70 will be explained.

The image storage apparatus (image saving section) 70 is equipped with a communication device and a large-capacity storage means such as a hard disk, and stores medical image data (image information) transmitted from the medical image data generating apparatus 10, the candidacy abnormal shadow detecting apparatus 30, etc. provided on the communication network, together with an attached bit of information, to form a data base. The image storage apparatus 70, having received a request of medical image data from any one of the apparatus provided on the communication network N, distributes the designated medical image data to the designated transmission end.

In addition, in this embodiment, the above-mentioned diagnosis section (candidacy abnormal shadow detecting apparatus 30) is equipped with a defect information detecting section which investigates attached bits of information stored and saved in the image storage apparatus (image saving section) concerned, and detects whether or not the above-mentioned information on the existence of a defect is present. If information indicating the existence of a defect is detected in this section, by an identification section provided in the diagnosis section, at least one of the input section information and the cassette information (both in this case) is detected and analyzed from the attached bit of information having said information indicating the existence of a defect recorded. Then, at least one of said input section, cassette, and stimulable phosphor sheet (in this case, the input section and cassette) is identified, and for example, a display indicating a notification to that effect is carried out by the output apparatus (in this case, a display output apparatus) 90 to be described later. By the practice of an investigation and maintenance operation for the input section, the cassette, etc. based on this, it is possible to efficiently remove a failure generating a defective image information.

Next, the output apparatus 90 will be explained.

The output apparatus (output section) 90 is equipped with a communication device and carries out the output of medical image data (image information) received through the communication network N.

As regards the output apparatus 90, a high-definition, high-brightness CRT or liquid crystal display dedicated to medical images, for example, a CRT (Cathode Ray Tube), a liquid crystal display, a plasma display, etc. can be employed for it.

Further, for the output apparatus 90, a film output apparatus can be employed. In this case, the output apparatus 90 applies an exposure processing to a film on the basis of image data received through the communication network N, to record the medical image data on the film.

Further, for the output apparatus 90, a printer or the like which forms an image on a recording medium such as a paper sheet on the basis of image data can be employed. In this case, the output apparatus 90 forms an image on a recording medium on the basis of image data received through the communication network N, and outputs said recording medium.

In addition, in this embodiment, any one of the above-mentioned output sections (output apparatus 90 (display output apparatus, film output apparatus, printer, etc.)) comprises an output section information recording section, and is provided with a function to record the information (output section information) on the output section (output apparatus 90 (display output apparatus, film output apparatus, printer, etc.)) used in the outputting (display, printing, etc.) of the image information in the header area of the above-mentioned medical image data (image information).

By this, in a case where there are a plurality of kinds of output section (for example, a display output apparatus, a film output apparatus, a printer, etc.), in a case where there are a plurality of apparatus for each kind, or in other cases like that, it is possible to identify the output section (the image storage apparatus 70 and the output apparatus 90 (the display output apparatus, film output apparatus, printer, etc.) having outputted (saved, displayed, printed, etc.) the image information concerned.

For example, it can be cited a case where individual numbers are previously attached to their respective output sections (their respective image storage apparatus 70 and their respective display output apparatus, film output apparatus, printer, etc. as the output apparatus 90), and the number concerned is recorded.

Further, in the case where a film output apparatus is employed for the output apparatus, it is also appropriate to record the kind, individual number, etc, of a photosensitive film to be used in the outputting as bits of film information in the output section information recording section.

Incidentally, for concrete examples of the header information to be recorded in the header area of medical image data (image information of a patient) or on a film together with the image information, such items as described below can be considered.

First, it is premised that the medical image processing system 100 comprises 100 sheets of plate (cassette) (numbers 1 to 100 are defined for the plate ID), 10 input sections (input apparatus) (numbers 1 to 10 are defined for the reader ID), 10 output sections (a plurality of kinds of output apparatus) (numbers 1 to 10 are defined for the imager ID).

Further, as regards the medical image generating apparatus (input section) 10, the image storage apparatus (output section) 70, and the output apparatus (output section) 90, it is also appropriate that maintenance operations for the apparatus are carried out and the version upgrading of the control software for repairing failures or upgrading the specification is also carried out even after the installment of the apparatus, and in the input section and output section, bits of information on the maintenance of apparatus, version upgrading of the control software, etc., which have been converted into numerals, are recorded in relation to the image information (as attached bits of information). For example, it is also appropriate that bits of status information such as information on the maintenance or version upgrading as described above are converted into numerals (numbers 1 to 10 are defined for the reader status of the input section (reader), and numbers 1 to 10 are defined for the imager status of the output section (imager)) and recorded. Further, it is also appropriate to make the above-mentioned film information indicating the kind, individual number, etc. of a photosensitive film to be used in the outputting included in the imager status. In this case, it is appropriate to convert the film information into a predetermined numeral for recording.

Further, it is appropriate that the above-mentioned numerals (numbers) are recorded in the order of, for example, "plate ID/reader ID/reader status/imager ID/imager status", that is, these are recorded in such an arrangement as "55/3/5/6/4", which are made to be the bits of header information.

Besides, it is possible that, among the above-mentioned items, concerning those not required, the numerals representing the items concerned are made to be "0" and the items themselves are left existing, but sometimes an item itself is deleted.

For example, in the case where the reader status is the same for all the readers, it is possible to make the header information such one as "plate ID/reader ID/imager ID/imager status" with the item of the reader status omitted. Further, there is also a case where the above-mentioned output section information, input section information, etc. are made up not of numerals but of signs.

Further, in the case where the output section is not an apparatus which practices the outputting on a film or the like (an apparatus which practices the outputting by display), it is also appropriate that only the ID is attached to the image information, detailed information is recorded in another recording apparatus, and the using of the information for perusal is enabled by the inputting of the ID.

Furthermore, in this embodiment, input section information and output section information are made to be recorded in the input section and the output section respectively; however, this invention is not limited to this, but it is also possible that the system is equipped (for example, in the output section) with an input/output section correlating section for collecting all the information, and the input section information and the output section information correlated here are recorded in the header area or the like (as attached bits of information) in the input/output section information recording section (provided in the output section, for example). In this case, it may be appropriate to further record the above-mentioned diagnosis result information in relation to those.

Besides, in the case where image information is recorded on a film in the output section, also the above-mentioned information in the header area is recorded on the film. Further, at this time, it is also appropriate to record the kind, individual number, etc. of the photosensitive film used as film information.

In addition, in cases as described in the above, by the outputting based on the same data by means of different imagers, it is enabled to immediately judge whether or not the problem is specific to the imager. As regards the imager having its cause of a failure clarified, it becomes possible to maintain the image quality by the practice of suitable maintenance operations, which is desirable.

Further, output apparatus generally require a maintenance operation, and the image quality is often improved after a maintenance operation; therefore, it is preferable to add information on the condition of the apparatus (condition about maintenance) on top of the individual ID of each apparatus.

Further, in cases where a viewer is used, it can be said that the above-mentioned thing is to be applied for every changing of the LUT used.

As explained up to now, by a medical image processing system of this embodiment, it is actualized that at least one of input section information enabling the identification of the input section (reader) used in the inputting and cassette information enabling the identification of the cassette or stimulable phosphor accommodated in the cassette loaded in the above-mentioned input section and used in the output of image information is recorded; therefore, in cases where a failure is generated in image information, it is possible to immediately judge whether or not the cause is in the input section and/or in the cassette by the mere confirmation of the input section and cassette concerned, and the pursuit of the cause of the failure can be carried out easily.

Further, in this embodiment, there is provided an image information confirming section such as a viewer for displaying image information for confirmation, and if it is discovered by the display for confirmation that there is a defect in image information (for example, disturbance of image information due to dusts etc.), information indicating the existence of a defect can be recorded as information attached to image information; therefore, it is possible to easily discriminate between normal image information and defective image information, and after that, the pursuit of the cause of the failure can be quickly carried out.

Further, in this embodiment, in cases where image information has a defect, at least one of the input section, cassette, or stimulable phosphor sheet which have been used in the inputting of the defective image information concerned, is automatically identified, the pursuit of the cause of the failure can be more simply carried out.

Furthermore, in this embodiment, image information is transmitted and received between the sections by means of a standard signal based on DICOM standard; therefore, the image information can cope with various kinds of input apparatus and output apparatus.

Further, by a medical image processing apparatus of this embodiment, it is actualized that, when image information is outputted to a photosensitive film in an output section such as an imager, at least one of input section information enabling the identification of the reader used in the inputting, output section information enabling the identification of the imager having carried out the outputting concerned, and film information on the photosensitive film used at the time of carrying out the outputting concerned is recorded; therefore, in cases where a failure of image information is generated, it is possible to immediately judge whether or not the cause is in the input section, output section, or film by the mere confirmation of the input section, output section, and film concerned, and the pursuit of the cause of the failure can be easily carried out.

Further, in this embodiment, it is actualized that, in the case where image information is outputted for display in the output section such as a viewer, input section information enabling the identification of the reader used in the inputting and output section information enabling the identification of the viewer used in the outputting concerned are correlated with each other and then recorded; therefore, in cases where a failure of image information is generated, it is possible to immediately judge whether or not the cause is in the input section or output section by the mere confirmation of the input section and output section concerned, and the pursuit of the cause of the failure can be easily carried out.

Further, in this embodiment, it is actualized that, in the case where image information is outputted for display in an output section such as a viewer and diagnosis is carried out on the basis of said image information, diagnosis result information, together with input section information and output section information, is also recorded in relation to the image information; therefore, in cases where a failure of image information is generated, it is possible to immediately judge where the cause is by the mere confirmation of the specified section, and the pursuit of the cause of the failure can be easily carried out.

Furthermore, in this embodiment, it is actualized that, when image information is inputted, input section information enabling the identification of the reader used in the inputting concerned is recorded as an attached bit of information, and when image information is outputted for display in an output section such as a viewer, output section information enabling the identification of the viewer used in the outputting concerned is further recorded as an attached bit of information; therefore, in cases where a failure of image information is generated, it is possible to immediately judge whether or not the cause is in the input section or output section by the mere confirmation of the input section and output section concerned, and the pursuit of the cause of the failure can be easily carried out.

Further, in this embodiment, it is actualized that, when image information is inputted, input section information enabling the identification of the reader used in the inputting concerned is recorded as an attached information, and when image information is outputted for display in an output section such as a viewer, output section information enabling the identification of the viewer used in the outputting concerned is recorded as an attached bit of information, while result information of the diagnosis carried out on the basis of image information is recorded as an attached bit of information; therefore, in cases where a failure of image information is generated, it is possible to immediately judge where the cause is by the mere confirmation of the specified section, and the pursuit of the cause of the failure can be easily carried out.

Further, in this embodiment, it is actualized that status information of an input section and that of an output section are recorded in relation to input section information and output section information; therefore, in cases where a failure of image information is generated, it is possible to immediately judge where the cause is more simply, and the pursuit of the cause of the failure can be easily carried out.

In addition, it is appropriate that the image processing includes gradation processing, frequency processing, correction processing of pixel size, correction processing of the number of gray levels, dynamic range compression processing (the publication of the unexamined patent application H3-222577), abnormal shadow detection processing (the publication of the unexamined patent application S62-125481), position adjustment processing for correcting a positional deviation of corresponding portions between a plurality of images (the publication of the examined patent application S61-14553, and the publication of the unexamined patent applications S63-278183 and H1-70236), frequency processing using a multi-resolution method (the publication of the unexamined patent application H11-345331), etc.

In addition to the above-mentioned, concerning the detailed structure and detailed operation of an medical image processing system of this embodiment also, it is possible to suitably modify them within a range not departing from the intention of this invention.

### (EMBODIMENT 2 OF THE INVENTION)

In the following, the embodiment 2 of this invention will be explained in detail with reference to the drawings. However, the scope of the invention is not to be limited to the examples shown in the drawings. Besides, in the following, as an example of the embodiment characterizing this invention, a medical image pickup system for carrying out radiographing at a site of round visits utilizing a portable terminal and a movable image pickup apparatus will be explained.

First, the structure of this embodiment will be explained.

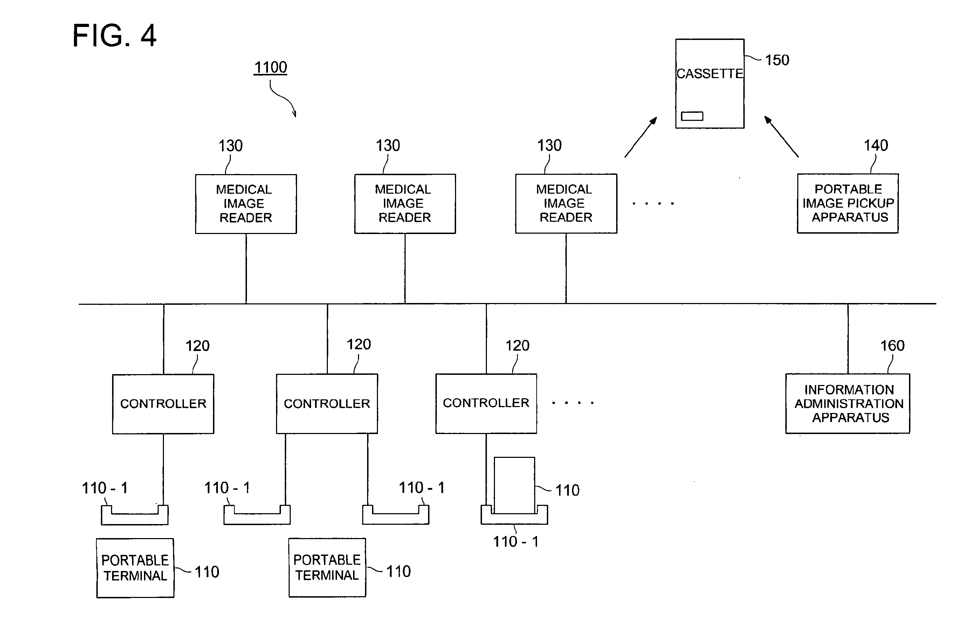

FIG. 4 is a conceptual drawing showing the system' structure of a medical image pickup apparatus 1100 of this invention. As shown in FIG. 4, the medical image pickup system 1100 is composed of portable terminals 110, communication terminals 110-1, control apparatus 120, medical image reading apparatus 130, a portable image pickup apparatus 140, a cassette 150, an information administration apparatus 160, etc. Further, the control apparatus 120, the medical image reading apparatus 130, and the information administration apparatus 160 are connected to one another through a network N, and the portable terminals 110 and their respective control apparatus 120 have a structure such that they can be connected with each other through the respective communication terminals 110-1.

The portable terminal 110 is an information terminal apparatus of a portable type carried by a radiological technologist (radiographer) who operates the portable image pickup apparatus 140, and carries out communication with the control apparatus 120 through the communication terminal 110-1 to be described later. Further, the portable terminal 110 acquires image pickup order information from the control apparatus 120 and displays it, and in a case where there are image pickup orders of plural patients, the portable terminal 110 retrieves and displays the image pickup order information corresponding to the desired patient ID out of the image pickup order information of the plural patients. Further, the portable terminal 110, when starting image pickup, acquires the identification information of the radiographer (hereinafter referred to as "radiographer ID"), the identification information of the cassette 150 (hereinafter referred to as "cassette ID"), and the identification information of the image pickup apparatus (hereinafter referred to as "image pickup apparatus ID, and stores them in correspondence with the image pickup order information.

The communication terminal 110-1 is connected with the control apparatus 120 through a cable or the like, and controls the transmission and reception of data between the portable terminal 110 and the control apparatus 120 connected to the communication terminal 110-1. For example, the communication terminal 110-1 controls the transmission of image pickup order information from the control apparatus 120 to the portable terminal 110, and controls the transmission of a radiographer ID, a cassette ID, and an image pickup apparatus ID from the portable terminal to the control apparatus 120.

The control apparatus 120 is an apparatus which receives image pickup order information from the information administration apparatus 160 and controls medical images on the basis of the received image pickup order information, and also controls image pickup history of medical images on the basis of information received from the portable terminal 110 and the medical image reading apparatus 130. For example, the control apparatus 120 transmits image pickup order information received from the information administration apparatus 160 to the portable terminal 110 through the communication terminal 110-1. Further, the control apparatus 120, after an image pickup has been finished, receives the radiographer ID, cassette ID, and image pickup apparatus ID brought in correspondence with the image pickup order information.

Further, the control apparatus 120 receives a medical image, the cassette ID brought in correspondence with the medical image, and the identification information of the reading apparatus (hereinafter referred to as "reading apparatus ID") from the medical image reading apparatus 130. Further, the control apparatus 120 controls a medical image, a radiographer ID, a cassette ID, an image pickup apparatus ID, a reading apparatus ID, etc. in correspondence with one another on the basis of image pickup order information. Further, the control apparatus 120 transmits a medical image, a radiographer ID, a cassette ID, an image pickup apparatus ID, and a reading apparatus ID to the information administration apparatus 160 in correspondence with image pickup order information.

The medical image reading apparatus 130 is a medical image reading apparatus for reading a medical image recorded in the cassette 150. The medical image reading apparatus 130 irradiates a stimulable phosphor sheet in the cassette 150 with excitation light, the stimulation light emitted from the sheet by the excitation light is photoelectrically converted, and the obtained image signal is subjected to A/D conversion, to become medical image data. Further, the medical image reading apparatus 130 reads the cassette ID attached to the cassette 150, and transmits the medical image data, the cassette ID, and the reading apparatus ID of its own apparatus to the control apparatus 120 in correspondence with one another.

The portable image pickup apparatus 140 is a movable medical image pickup apparatus, and carries out an image pickup of a patient at a site of round visits, to record a medical image in the cassette 150 which is capable of being mounted and dismounted in the apparatus mainframe. The cassette 150 has a built-in stimulable phosphor sheet for accumulating a part of radiation energy, and accumulates a part of radiation energy having emitted from the radiation source and passed a test object disposed between the radiation source and the cassette in the above-mentioned stimulable phosphor sheet. In addition, on the surface of the cassette 150, a bar code or the like for indicating the cassette ID standing for the identification information of the cassette 150.

The information administration apparatus 160 is a terminal for collectively control a plurality of sets of image pickup order information designated by a doctor; in response to the requirement instruction from the control apparatus 120, it extracts a set of image pickup order information, which is transmitted by it to the control apparatus 120. Besides, as regards another information administration apparatus, a reception apparatus (not shown in the drawing) for carrying out the reception of booking of image pickup orders may be employed; that is, it is also appropriate to employ an information control system such as an HIS (Hospital Information System) and an RIS (Radiology Information System).

For the network N, various types of line such as a LAN (Local Area Network), a WAN (Wide Area Network), and the Internet can be employed. Besides, a wireless communication or an infrared communication may be employed so long as it is permitted for the use in a medical treatment organization such as a hospital; however, because important information of a patient is included in the transmission and reception of image pickup order information, it is desirable to make image pickup order information a cipher.

Next, apparatus which are regarded as principal structural elements of this invention will be explained in detail.

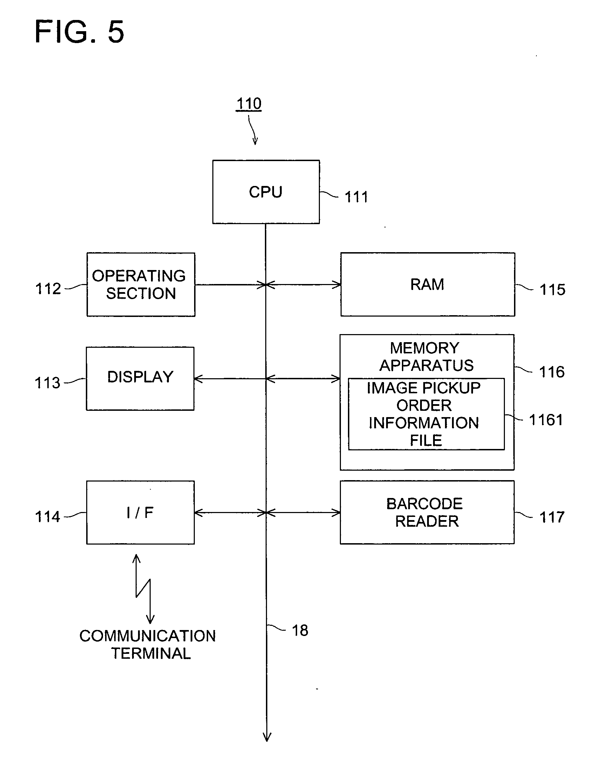

FIG. 5 is a block diagram showing the functional structure of the portable terminal 110. As shown in FIG. 5, the portable terminal 110 is composed of a CPU 111, an operation section 112, a display 113, an I/F 114 as a communication device, a RAM 115, a storage device 116 as a storage means, a bar code reader 117 as an acquisition means, etc., and all the sections are connected one another through a bus 118.

The CPU (Central Processing Unit) 111 runs a program which is designated out of a system program and various kinds of application program stored in the storage device 116 on the RAM 115, and controls every unit of the portable terminal 110 in accordance with said program.

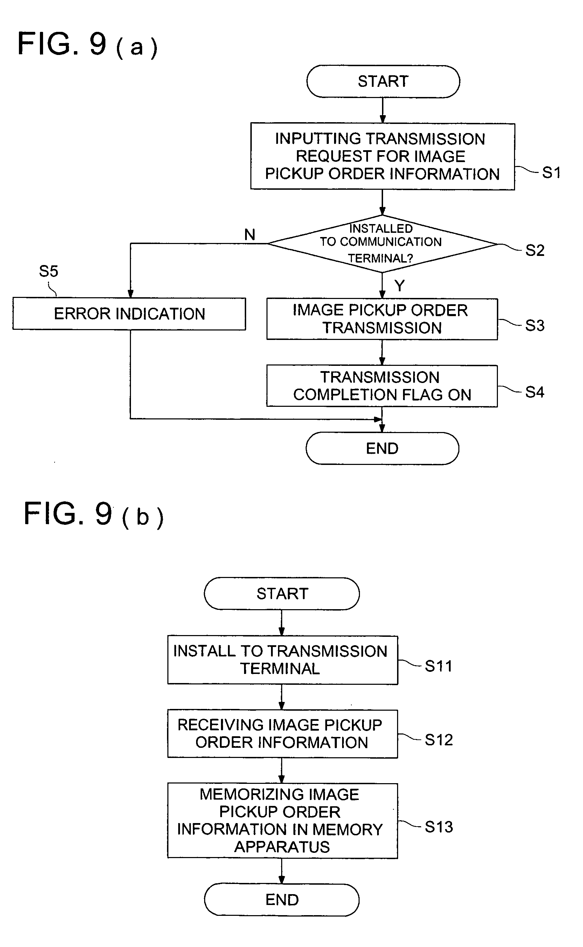

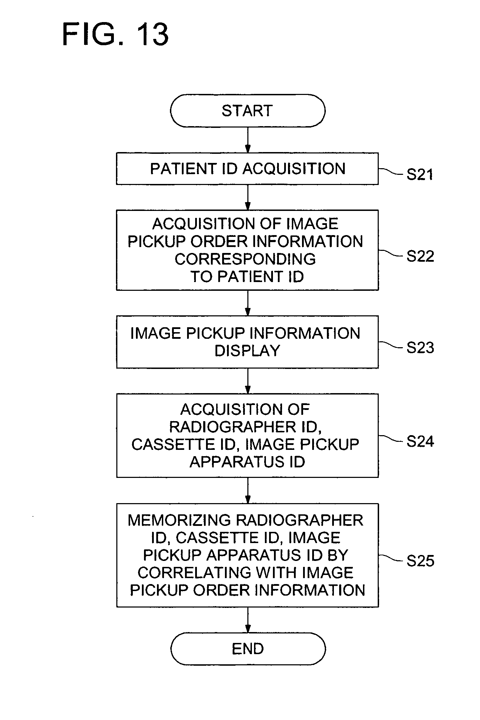

To state it concretely, the CPU 111 reads out an image pickup preparation processing program and an image pickup start processing program from the storage device 116, and practices an image pickup preparation processing (refer to FIG. 9(b)) and an image pickup start processing (refer to FIG. 13) to be described later. In addition, the detail of the processings will be described later.

The operation section 112 is equipped with a cursor key, numeral keys, various kinds of functional key, etc., and outputs a depress signal corresponding to a key depressed by the radiographer to the CPU 111. Further, the operation section 112 is equipped with a jog-dial key, and outputs an instruction signal for scrolling (moving) the information displayed on the display 113 to the CPU 111 in response to the operation of the jog-dial key. Further, when the jog-dial key is depressed, it outputs a depress signal for the displayed information to the CPU 111. In addition, the operation section 112 may be equipped with a pointing device such as a touch panel, or some other input device as occasion demands.

The display 113 is a display means equipped with a display based on an LCD (Liquid Crystal Display) or the like, and displays image pickup order information and various kinds of information such as a patient ID, a radiographer ID, a cassette ID, an image pickup apparatus ID acquired on the basis of display instruction from the CPU 111.

The I/F 114 is an interface for connecting the portable terminal 110 with the communication terminal 110-1, and when the portable terminal 110 is connected to the communication terminal 110-1, it outputs a detection signal to the CPU 111. Further, the I/F 114 carries out the adjustment of data transfer speed and the transformation of data format between the portable terminal 110 and the control apparatus 120 through the communication terminal 110-1, to mediate between both the apparatus for give-and-take of data.

For example, the I/F 114 receives image pickup order information from the control apparatus 120, and after the end of an image pickup operation, it also transmits the radiographer ID, cassette ID, and image pickup apparatus ID brought in correspondence with the image pickup order information to the control apparatus 120. In addition, the I/F 114 has a structure such that it has a cellular phone terminal such as a PHS connected with it, and establishes a wireless communication system to carry out transmission and reception of data.

The RAM (Random Access Memory) 115 has a work memory area for storing the above-mentioned designated application program, an input instruction, input data, the result of processing, etc.

The storage device 116 comprises a storage medium (not shown in the drawing) having programs and data previously stored, and this storage medium stores a system program, various kinds of application program corresponding to said system program, and data obtained by the processing by various kinds of processing program. Further, this storage medium is made up of a magnetic or optical storage medium or a semiconductor memory, and is fixedly provided in the storage device 116 or is mounted in such a way as to be capable of being mounted or dismounted.

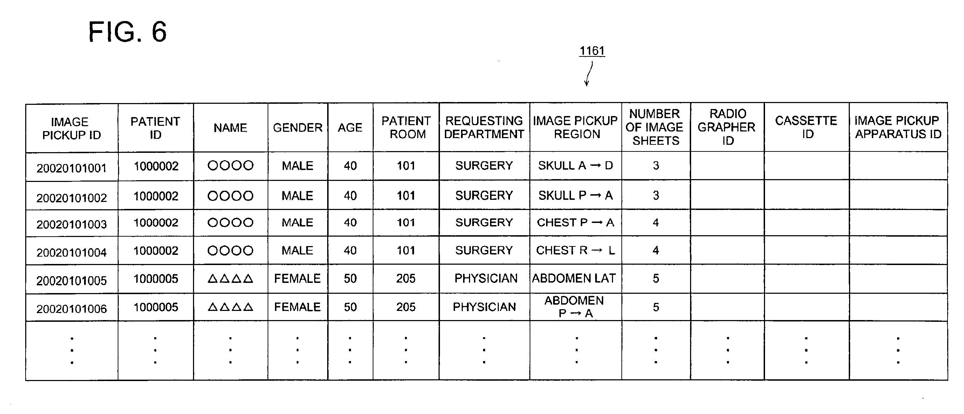

Further, the storage device 116 stores an image pickup order information file 1161 for storing image pickup order information received from the control apparatus 120. The image pickup order information file 1161 will be explained with reference to FIG. 6. FIG. 6 is a drawing showing an example of the data structure of the image pickup order information file 1161. As shown in FIG. 6, the image pickup order information file 1161 has items for storing the image pickup ID, patient ID, name, sex, age, patient room, requesting department, image pickup region, number of image sheets, and items for storing the radiographer ID, cassette ID, image pickup apparatus ID which are acquired at the start of image pickup, and stores data corresponding to each of the items for each image pickup order information.

In the item of image pickup ID, identification codes allotted uniquely for the identification of their respective image pickup works (for example, 20020101001, 20020101002, 20020101003, ---) are stored. In the item of patient ID, identification codes allotted uniquely for the identification of the respective patients to be subjected to image pickup (for example, 10000002, 10000005, ---) are stored. In the item of name, bits of character information indicating the name of the respective patients to become the object of image pickup are stored, and in the item of sex, bits of character information indicating the sex of the respective patients to become the object of image pickup are stored. In the item of age, bits of numeral information indicating the age of the respective patients to become the object of image pickup are stored, and in the item of patient room, bits of character information indicating the respective patient rooms to become the image pickup site are stored.

In the item of requesting department, bits of character information indicating the respective requesting departments having requested image pickup are stored, in the item of image pickup region, bits of information indicating the respective image pickup regions (for example, skull A → P, skull P → A, chest P → A, ---) are stored, and in the item of number of image pickup sheets, the number of sheets of the respective medical images to be radiographed are stored as bits of numeral information (for example, 3, 4, 5, ---).

In the item of radiographer ID, identification codes uniquely allotted for the identification of the respective radiographers having practiced the image pickup work (for example, bits of information to be read from the bar code attached to the ID card or the like owned by the respective radiographers) are stored. In the item of cassette ID, identification codes uniquely allotted for the identification of the respective cassettes having been used in the image pickup operation (for example, bits of information to be read from the bar code or the like attached to the respective cassettes 150) are stored. In the item of image pickup apparatus ID, identification codes uniquely allotted for the identification of the respective portable image pickup apparatus 140 having practiced the image pickup process (for example, bits of information to be read from the bar code or the like attached to the respective portable image pickup apparatus 140).

In addition, the data of the above-mentioned radiographer ID, cassette ID, and image pickup apparatus ID are not stored in the image pickup order information file 1161 before the image pickup process, and the data for the above-mentioned items read by the bar code reader 117 at the start of the image pickup process are made to be stored.

Further, it is also possible to make the image pickup order information have such a structure as to store, in addition to the patient ID, name, sex, and age for the patient information, various kinds of patient information such as the name of the doctor in charge of the patient, warning information to make warning for an infectious disease, being allergic to medical substances or not, being pregnant or not, additional medical history, necessity of a special care such as a wheel chair or a stretcher, the name of clinical diagnosis, and secrecy matters. Further, also it is appropriate to make the image pickup order information have such a structure as to store, in addition to the image pickup region, image pickup apparatus, number of image pickup sheets for the image pickup information, various kinds of image pickup information such as the method of radiographing (simple radiographing, contrast medium radiographing, etc.), and planned image pickup date.

The bar code reader 117 is an example of an acquisition means for_acquiring a radiographer ID, cassette ID, image pickup apparatus ID, etc., and is equipped with a scanner, which is an optical reading device. The bar code reader 117 acquires information indicated by a bar code through reading a bar code by the scanner and decoding it in accordance with a specified standard. For example, the bar code reader 117 reads a barcode attached to an ID card or the like of a radiographer, and acquires the radiographer ID when an image pickup operation is carried out, and reads a bar code attached to the cassette 150 for recording a medical image, and acquires the cassette ID.

Further, the bar code reader 117 reads a bar code attached to the bed side of a patient or a part of the body of a patient, and acquires the patient ID; further, it reads a bar code attached to an image pickup apparatus, and acquires the image pickup apparatus ID. In addition, the term "specified standard" means JAN code, UPC code, CODE39, CODE93, CODE128, NW-7, INDUSTRIAL 2 of 5, ITF physical distribution code, etc.

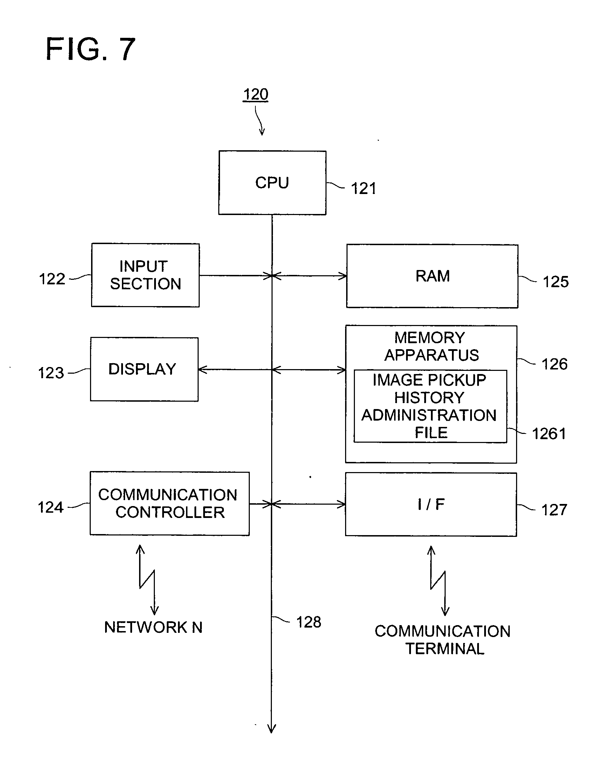

FIG. 7 is a block diagram showing the functional structure of the control apparatus 120. As shown in FIG. 7, the control apparatus 120 is equipped with a CPU 121, an input section 122, a display 123, a communication controller 124, a RAM 125, a memory apparatus 126, an I/F 127, etc., and all the sections are connected with a bus 128.

The CPU 121 reads out a system program and various kinds of control program stored in the storage device 126, runs a program on the RAM 125, and makes a centralized control of the operation of every section in accordance with said control program. Further, the CPU 121 practices various kinds of processing in accordance with a program developed on the RAM 125, makes the RAM 125 store the processing result, and makes the display 123 display it.

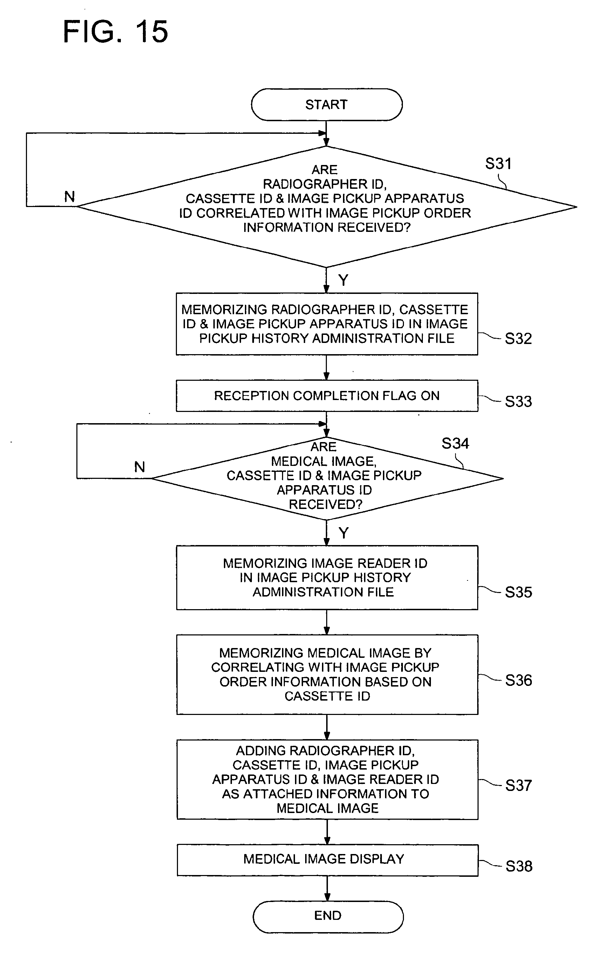

To state it concretely, the CPU 121 reads out an image pickup preparation processing program and a post-image pickup processing program from the storage device 126, and practices an image pickup preparation processing (refer to FIG. 9(a)) and a post-image pickup processing (refer to FIG. 15) to be described later.

The input section 122 includes a key board equipped with a cursor key, numeral keys, various kinds of functional key, etc., and outputs a depress signal corresponding to a key depressed in this key board to the CPU 121. Further, it is also appropriate that the input section 122 is equipped with a pointing device such as a mouse or a touch panel, or with some other input device as occasion demands.

The display 123 is made up of an LCD, a CRT (Cathode Ray Tube), or the like, and displays an input instruction, data, etc. received from the input section 122 in accordance with an instruction based on a display signal inputted from the CPU 121.

The communication control section 124 is made up of a LAN adapter, a router, a TA (Terminal Adapter), or the like, and controls the communication between the apparatus connected to the network N through a dedicated line or a communication line such as an ISDN line.

The RAM 125 has a storage area formed for temporarily storing a system program and a control program, which are practicable by the CPU 121 and read out from the storage device 126, input or output data, parameters, etc., in the various kinds of processing to be practiced and controlled by the CPU 121.

The storage device 126 is made up of an HDD (Hard Disk Drive), a non-volatile semiconductor memory, or the like, and stores a system program, various kinds of processing program corresponding to said system program to be practiced by the CPU 121, results of processing, etc. Further, it comprises a storage medium (not shown in the drawing) having programs and data previously stored; this storage medium is made up of a magnetic or optical storage medium or a semiconductor memory, and is fixedly provided in the storage device 126 or is mounted in such a way as to be capable of being mounted or dismounted. These various kinds of program are stored in a form of a readable program code, and the CPU 121 practices successively the operations in accordance with the program code concerned.

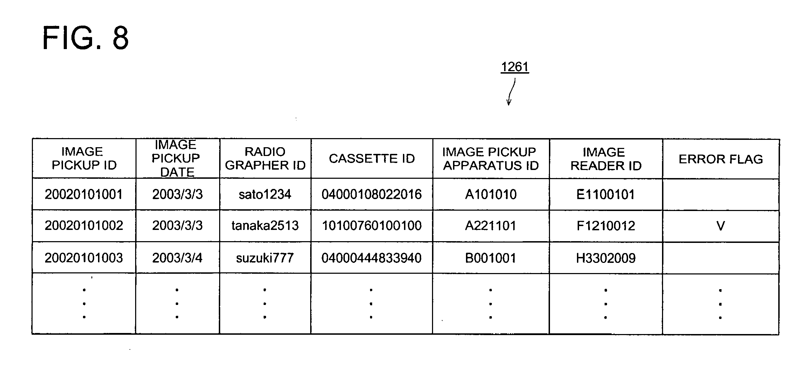

Further, the storage device 126 stores an image pickup order information file (not shown in the drawing) for storing image pickup order information received from the information administration apparatus 160 and an image pickup history administration file 1261 for controlling the image pickup history of a medical image. In addition, because the image pickup order information file has approximately the same structure as the above-mentioned image pickup order information file 1161 (refer to FIG. 6), its drawing and detailed explanation will be omitted. With reference to FIG. 8, the image pickup history administration file 1261 will be explained.

FIG. 8 is a drawing showing an example of the data structure of the image pickup history administration file 1261. As shown in FIG. 8, in the image pickup history administration file 1261, there are provided items for storing an image pickup ID, an image pickup date, a radiographer ID, a cassette ID, an image pickup apparatus ID, a reading apparatus ID, and an error flag.

In the item of image pickup ID, identification codes uniquely identifying the respective bits of image pickup order information stored in the above-mentioned image pickup order information file 1161 (for example, 20020101001, ---) are stored, and by the image pickup ID, data stored in other items are brought into correspondence with the above-mentioned image pickup order information. In the item of image pickup date, bits of information indicating the date when the respective medical images were radiographed (for example, 2003/3/3, ---) are stored.

In the item of radiographer ID, bits of identification information uniquely identifying the respective radiographers having radiographed the respective medical images (for example, sato1234, ---) are stored, and in the item of cassette ID, bits of identification information uniquely identifying the respective cassettes having stored the respective medical images (for example, 04000108022016, ---) are stored. In the item of image pickup ID, bits of identification information uniquely identifying the respective image pickup apparatus or the respective portable image pickup apparatus 140 (for example, A101010, ---) are stored.

In the item of reading apparatus ID, bits of identification information uniquely identifying the respective medical image reading apparatus 140 having read the respective medical images from the respective cassettes 150 (for example, E1100101, ---) are stored, and in the item of error flag, check flags for indicating whether or not an error is generated in the respective medical images.

In addition, the above-mentioned image pickup ID, image pickup date, radiographer ID, cassette ID, and image pickup apparatus ID are information transmitted from the portable terminal 110 in the post-image pickup processing to be described later, and the reading apparatus ID is information transmitted from the medical image reading apparatus 140. Further, the information on whether or not an error flag has been checked is information inputted by the radiographer in the post-image pickup processing. Besides, the kinds of information to be stored in the image pickup history administration file 1261 are not limited to the above-mentioned examples, and it is possible to store various kinds of information other than those as occasion demands.

The I/F 127 is an interface for connecting the control apparatus 120 with the communication terminal 110-1, and when it is detected that the portable terminal 110 has been connected with the communication terminal 110-1, it outputs a detection signal to the CPU 111. Further, the I/F 127 carries out the adjustment of data transfer speed and the transformation of data format between the control apparatus 120 and the portable terminal 110 through the communication terminal 110-1, to mediate both the apparatus for give-and-take of data. For example, the I/F 127 transmits image pickup order information to the portable terminal 110 before the start of an image pickup process, and also receives the radiographer ID, cassette ID, and image pickup apparatus ID having been brought into correspondence with the image pickup order information from the portable terminal 110 after the completion of the image pickup process.

Next, the medical image reading apparatus 130 will be explained. In addition, because the functional structure of the medical image reading apparatus 130 is approximately the same as that of the above-mentioned control apparatus 120, the corresponding signs are attached to the same parts and the drawings and detailed explanation will be omitted. That is, the medical image reading apparatus 130 is equipped with a CPU 131, an input section 132, a display section 133, a communication controller 134, a RAM 135, an image reading section 137, a bar code reader 138, etc., and all the sections are connected with one another through a bus 139.

The image reading section 137 irradiates a stimulable phosphor layer in the cassette 150 by excitation light to make it emit fluorescent light corresponding to the radiation energy accumulated, and photoelectrically converts the emitted fluorescent light; thus, radiation image data are acquired.

The bar code reader 138 is an example of an acquisition means for acquiring a cassette ID, and is equipped with a scanner which is an optical reading device. The bar code reader 138 reads a bar code attached to the surface of the cassette 150 loaded in the image reading section 137, and acquires the cassette ID.

The CPU 131 acquires a medical image read by the image reading section 137, a cassette ID read by the bar code reader 138, and a reading apparatus ID identifying uniquely the medical image reading apparatus having carried out the reading of the medical image, that is, its own apparatus, controls the communication control section 134, and brings the cassette ID and the reading apparatus ID in correspondence with the medical image, to transmit these to the control apparatus 120. In this case, it is desirable that the cassette ID and reading apparatus ID which have been brought in correspondence with the medical image are transmitted on the basis of DICOM (Digital Imaging and Communications Medicine) standard, for example.

Next, the operation of a system of this embodiment will be explained.

In addition, a program for actualizing each of the functions described in the flow chart to be described later is stored in the storage device 116 of the portable terminal 110, in the storage device 126 of the control apparatus 120, or in the storage device 136 of the medical image reading apparatus 130 in a format of a program code capable of being read by a computer, and the CPU 111 of the portable terminal 110, the CPU 121 of the control apparatus 120, or the CPU 131 of the medical image reading apparatus 130 successively practices the operations in accordance with the program code concerned.

First, as a preparation processing for the practice of an image pickup process, an image pickup preparation processing to make the portable terminal acquire image pickup order information from the control apparatus 120 will be explained.

FIG. 9(a) is a flow chart showing an image pickup preparation processing to be practiced by the CPU 121 of the controller 120. As shown in FIG. 9(a), when an instruction for the transmission of image pickup order information is inputted by a radiographer through the input section 122 (step S1), the CPU 121 controls the I/F 127 to judge whether or not the portable terminal 110 is connected with the communication terminal 110-1 (step S2). Now, if the portable terminal 110 is not connected with the communication terminal 110-1 (step S2: NO), the CPU 121 carries out an error display on the display 123, and this image pickup preparation processing is finished.

On the other hand, if the portable terminal 110 is connected with the communication terminal 110-1 (step S2: YES), the CPU 121 acquires the image pickup order information from the storage device 126, and transmits the image pickup order information to the portable terminal 110 through the communication terminal 110-1 (step S3) Then, the CPU 121 sets to ON the transmission flag of the image pickup order information having been transmitted (step S4), to complete this image pickup order information transmission processing.

FIG. 9(b) is a flow chart showing an image pickup preparation processing to be practiced by the CPU 111 of the portable terminal 110. As shown in FIG. 9(b), the portable terminal 110 is connected with the communication terminal 110-1 in order to acquire an image pickup order information (step S11).

Subsequently, the CPU 111 controls the I/F 114, to receive image pickup order information transmitted from the control apparatus 120 (step S12). Then, the CPU 111 makes the image pickup order information file 1161 in the storage device 116 store the received image pickup order information (step S13), to finish this image pickup order information acquisition processing.

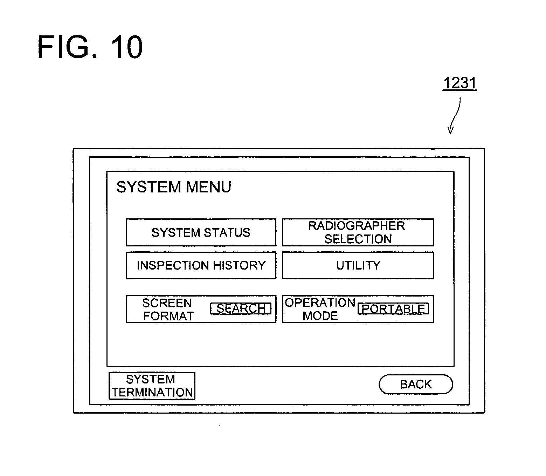

The display screen to be displayed on the display 123 of the control apparatus 120 in the above-mentioned image pickup preparation processing will be explained with reference to FIG. 10 to FIG. 12(b).

FIG. 10 is a drawing showing a menu screen 1231 for selecting a desired processing. As shown in FIG. 10, in the menu screen 1231, there are provided designation buttons for designating the menus "System state", "Selection of radiographer", "Inspection history", "Utility", "Screen format", and "Operation mode" respectively. Further, at each of the designation buttons for the "Screen format" and "Operation mode", an input item is provided; for example, when "portable" is inputted in the input item for the "Operation mode" and the button concerned is designated, the portable mode screen for the case where an image pickup process is carried out by the portable image pickup apparatus 140 is displayed. Further, when "normal" is inputted in the input item for this "Operation mode", the normal mode screen for the case where an image pickup process is carried out in a normal image pickup room is displayed.

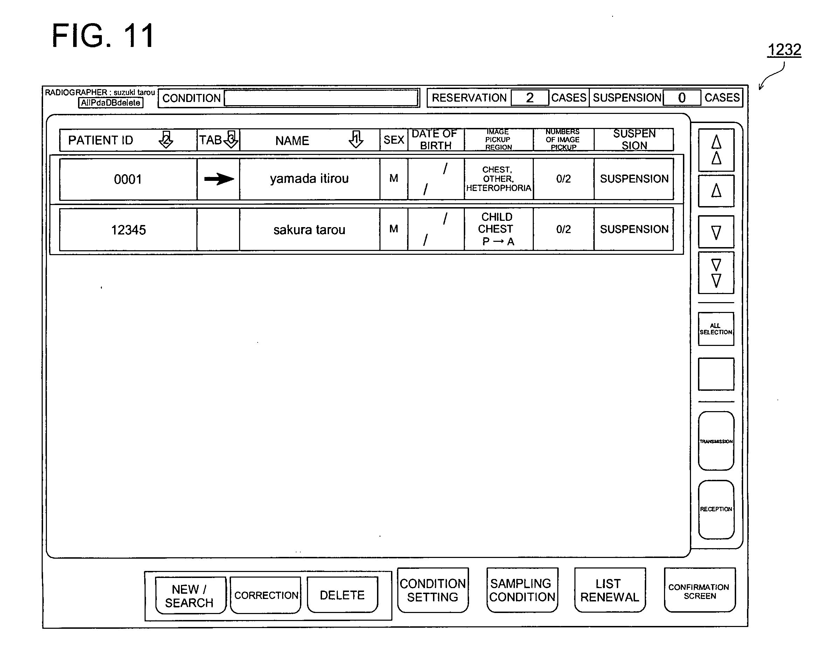

FIG. 11 is a drawing showing a portable list screen 1232 on which image pickup order information is displayed as an overall list. As shown in FIG. 11, in the portable list screen 1232, an area for displaying image pickup order information and an area for displaying designation buttons for inputting various kinds of designation are provided. In the area for displaying image pickup order information, as a part of image pickup order information, there are provided items for displaying the patient ID, tab, name, sex, date of birth, image pickup region, number of image pickup sheets, and suspension, and in each of the items, the corresponding data is displayed.

Further, near the right edge and lower edge of the portable list screen 1232, in the area for displaying designation buttons for inputting various kinds of designation, in addition to a selection key, there are provided designation buttons having respective text data of "transmission", "reception", "new/search", "correction", "delete", ---, "confirmation screen" displayed, and by each of the designation buttons being operated through the input section 122, the corresponding designation is inputted. For example, when the image pickup order information displayed on the portable list screen 1232 is selected by the selection key, and "transmission" key is operated, the selected image pickup order information is transmitted to the portable terminal 110. Now, when the image pickup order information is transmitted to the portable terminal 110, the transmission completion flag for the image pickup order information concerned is set to ON, and in the item of "tab", the check flag for the completion of transmission "→" is displayed. Further, when the designation button "new/search" is operated, the input screens 1233 and 1234 for newly registering image pickup order information are displayed.

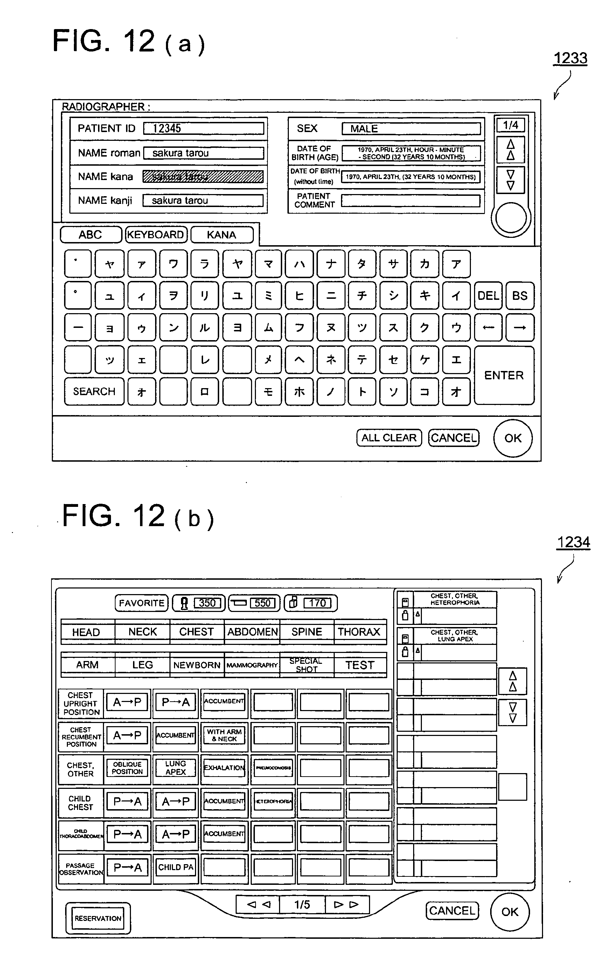

FIG. 12(a) and FIG. 12(b) are drawings showing the input screens 1233 and 1234 to be displayed in the case where the "new/search" designation button is operated in the above-mentioned portable list screen 1232. FIG. 12(a) is a drawing showing the input screen 1233 for newly inputting patient information in image pickup order information. As shown in FIG. 12(a), in the input screen 1233, there are provided an area for inputting patient information and an area for displaying character keys for carrying out character input.

In the area for inputting patient information displayed in the upper area of the input screen 1233, there are provided items for inputting the patient ID, name of the patient (romaji, kana, kanji), sex, date of birth, and comment, and an inputted data is to be displayed in the corresponding item in response to the operation of the input section 122. Further, in the area for displaying the character keys displayed in the lower area of the input screen 1233, the key input corresponding to the displayed character key concerned is carried out through a mouse or a touch panel provided in the input section 122. In addition, it is also possible to carry out the key input through a key board provided in the input section 122.

FIG. 12(b) is a drawing showing the input screen 1234 for inputting image pickup information out of a set of image pickup order information. As shown in FIG. 12(b), in the image pickup information input screen 1234, there are provided an area for displaying designation buttons each designating an image pickup region as an image pickup condition, an area for displaying designation buttons each designating an image pickup direction in an image pickup region, and an area for displaying the inputted image pickup region and image pickup direction.

In the area for displaying the designation buttons for image pickup region displayed in the upper left part of the input screen 1234, there are provided designation buttons having text data of, for example, "head", "neck", ---, and "TEST" displayed respectively, and by each of the designation buttons being designated through the input section 122, the corresponding image pickup region is selected. Further, in the area for displaying the designation buttons for image pickup direction displayed in the middle left part of the input screen 1234, half-tone line meshing displays are made, and these cannot be selected in the portable mode. That is, the half-tone line meshing indicates that the image pickup region to be displayed in said area is an image pickup region incapable of being radiographed by the portable image pickup apparatus 140. In addition, as a method of displaying non-selectable regions, in addition to the half-tone line meshing, it is possible to make them non-display or non-active display. Further, it is also appropriate to output a warning by a voice or an image, in the case where a non-selectable designation button is designated.

Further, in the area for displaying the designation buttons of image pickup direction displayed in the lower left part of the input screen 1234, there are provided designation buttons having text data of "oblique position", ---, "pneumoconiosis" displayed respectively as the image pickup directions in the image pickup region "chest, other" for example, and by each of the designation buttons being designated through the input section 122, the corresponding image pickup direction is selected. Further, in the area for displaying the image pickup region and image pickup direction displayed in the rightward part of the input screen 1234, an image pickup region and image pickup direction selected by one of the above-mentioned designation buttons being designated is displayed as "chest, other; oblique position", for example.

Next, it will be explained an image pickup start processing in which, in a patient room where an image pickup process is to be carried out, before the start of the image pickup process, an operation to bring the patient ID in correspondence with the image pickup order information is carried out, and the radiographer ID, cassette ID, and image pickup apparatus ID are stored in correspondence with the image pickup order information.

FIG. 13 is a flow chart showing an image pickup start processing to be practiced by the CPU 111 of the portable terminal 110. As shown in FIG. 13, the CPU 111 controls the bar code reader 117, and reads a bar code attached to the bed side of the patient or to a part of the patient body, to acquire the patient ID (step S21). Subsequently, the CPU 111 acquires from the storage device 116 the image pickup order information corresponding to the patient ID having been read (step S22).

Subsequently, the CPU 111 makes the display 113 display the acquired image pickup order information (step S23), while it controls the bar code reader 117, and reads the bar codes attached to the radiographer practicing the image pickup operations, the cassette 150, and the portable image pickup apparatus 140 respectively, to acquire the radiographer ID, the cassette ID, and the image pickup apparatus ID (step S24). Then, the CPU 111 makes the storage device 116 store the acquired radiographer ID, cassette ID, and image pickup ID in correspondence with the image pickup order information (step S25), to complete this image pickup start processing.

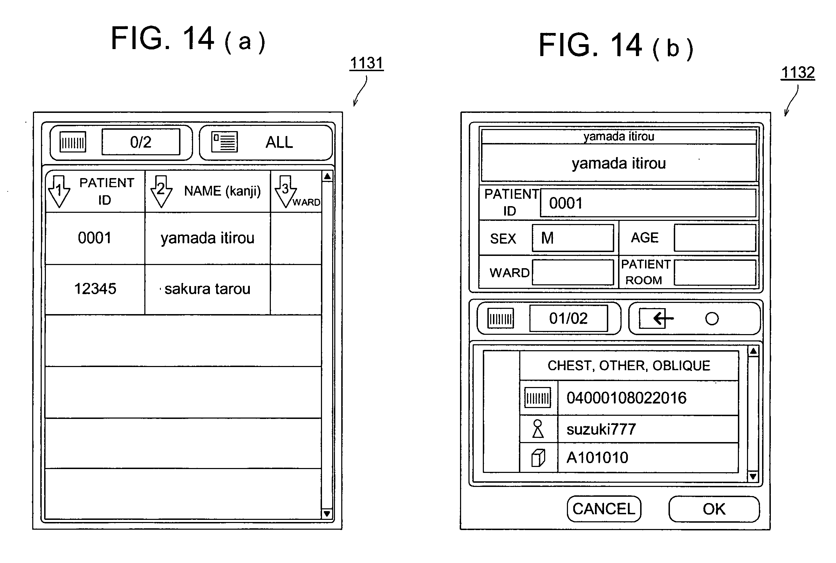

It will be explained with reference to FIG. 14(a) and FIG. 14(b), a display screen displayed on the display section of the portable terminal 110 in the above-mentioned image pickup start processing. FIG. 14(a) is a drawing showing a patient list screen 1131 for displaying the patients registered in the portable terminal 110 as an overall list. As shown in FIG. 14(a), in the patient list screen 1131, there is provided an area indicating patient information, and in this area, there are provided items for displaying a patient ID, a patient name, and a ward respectively. To state it concretely, in the item of patient ID, a numeral data "0001" is displayed, and in the item of name, a text data "Yamada Ichiro (in kanji)" is displayed.

FIG. 14(b) is a drawing showing a display screen 1132 for displaying image pickup order information of the corresponding patient in the case where a patient ID is acquired by the bar code reader 117 of the portable terminal 110. As shown in FIG. 14(b), in the display screen 1132, there are provided an area for displaying patient information and an area for displaying image pickup information. In the area for displaying patient information, there are provided items for displaying a patient ID, sex, age, ward, patient room respectively, and in each of the items, the corresponding data is displayed.

Further, in the area for displaying image pickup information, there are provided items for displaying an image pickup region, cassette ID, radiographer ID, image pickup apparatus ID respectively, and in each of the items of cassette ID, radiographer ID, image pickup apparatus ID, corresponding data acquired by the bar code reader 117 of the portable terminal 110 is displayed at the same time of the reading. To state it concretely, in the item of image pickup region, a text data "chest, other; oblique position" is displayed, and in the item of cassette ID, a numeral data "04000108022016" is displayed. Further, in the item of radiographer ID, a text-numeral data "suzuki777" is displayed, and in the item of image pickup apparatus ID, a text-numeral data "A101010" is displayed.

Subsequently, it will be explained a post-image pickup processing in which, after the completion of an image pickup process, in the control apparatus 120, the radiographer ID, cassette ID, and image pickup apparatus ID having been brought in correspondence with the image pickup order information of the finished image pickup process are acquired from the portable terminal 110, and also the medical image, cassette ID, and reading apparatus ID having been read from the medical image reading apparatus 130 are acquired.

FIG. 15 is a flow chart showing a post-image pickup processing to be practiced by the CPU 121 of the control apparatus 120. As shown in FIG. 15, when the CPU 121 receives the radiographer ID, cassette ID, and image pickup ID having been brought in correspondence with the image pickup order information of the finished image pickup process from the portable terminal 110 (step S31; YES), it makes the image pickup history administration file 1261 store the radiographer ID, cassette ID, and image pickup apparatus ID in correspondence with the image pickup order information (step S32). Then, the CPU 121 sets the reception finish flag to ON for the image pickup order information correlated with the received radiographer ID, cassette ID, and image pickup ID (step S33).

Subsequently, when the CPU 121 receives the medical image, cassette ID, reading apparatus ID from the medical image reading apparatus 130 (step S34; YES), on the basis of the cassette ID, the CPU 121 makes the image pickup history administration file 1261 store the received reading apparatus ID in correspondence with the image pickup order information (step S35). Further, the CPU 121 makes the storage device 126 store the medical image and the image pickup order information in correspondence with each other, on the basis of the cassette ID having been brought in correspondence with the medical image and the cassette ID having been brought in correspondence with the image pickup order information (step S36).

Further, the CPU 121 attaches the radiographer ID, cassette ID, image pickup apparatus ID, and reading apparatus ID corresponding to the medical image to the medical image concerned as attached bits of information (step S37). In the above, it is desirable that the attached bits of information to be attached to the medical image are attached in accordance with DICOM standard. Then, the CPU 121 makes the display 123 display the received medical image (step S38), to finish this post-image pickup processing.

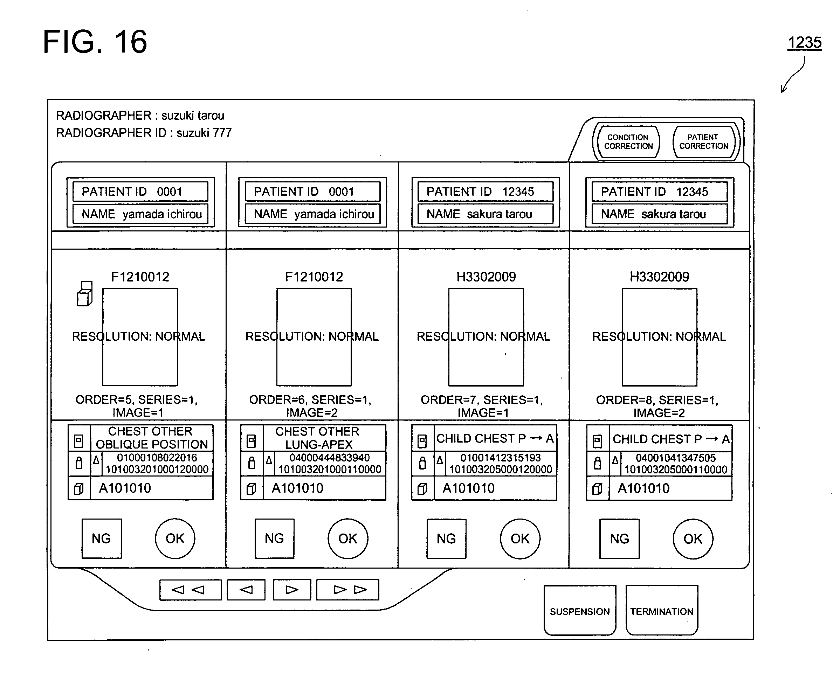

With reference to FIG. 16, a medical image to be displayed on the display 123 of the control apparatus 120 will be explained. FIG. 16 is a drawing showing a portable processing screen 1235 to be displayed on the display 123 in the case where an image pickup process is carried out by the utilization of the portable terminal 110. As shown in FIG. 16, in the portable processing screen 1235, medical images of a plurality of patients registered in the same portable terminal 110 are displayed on the same screen.

That is, in the portable processing screen 1235, there are provided an area for displaying radiographer information and an area for displaying patient information, a medical image, image pickup information for each patient, and four medical images can be displayed in one and the same screen. To state it concretely, in the area for displaying radiographer information lying in the upper left part of the portable processing screen 1235, there are provided an item for displaying a text data of "Suzuki Taro (in kana)" as the name of a radiographer, and an item for displaying a text-numeral data "suzuki777". Further, as regards the image to be displayed for each of the patients, to explain the medical image displayed at the leftward side, in the area for displaying the patient information, there are provided an item for displaying a numeral data of "0001" as the patient ID, and an item for displaying a text data of "Yamada Ichiro (in kana)" as the name of the patient.

In the lower part of the above-mentioned items, an area for displaying a medical image is provided, and there are provided a medical image, an item for displaying a numeral data of "F1210012" as the reading apparatus ID, and an item for displaying a text data of "normal" as the resolution. Further, under these, an area for displaying image pickup information is provided, and there are provided an item for displaying a text data of "chest, other; oblique position" as the image pickup region, an item for displaying a numeral data of "01000108022016" as the cassette ID, and an item for displaying a text-numeral data of "A101010" as the image pickup apparatus ID.

Further, in the lower part of the area for displaying image pickup information, there are designation buttons for inputting "NG" and "OK" respectively, by each of which the presence or absence of an abnormality of a displayed medical image is inputted. That is, in cases where an abnormality is generated in a medical image, when the "NG" designation button is designated through the input section 122, the error flag of the image pickup history administration file 1261 of the corresponding medical image is set to ON, to be stored as error information.

Besides, on the basis of the image pickup history administration file 1261, the control apparatus 120 can make a display as an overall list of the bits of identification information such as a radiographer ID, cassette ID, image pickup apparatus ID, and reading apparatus ID for medical images whose error flag have been set to ON. At this time, for example, it is possible to sort medical images having an abnormality generated for each of the bits of identification information and display them. By this, it is possible that a radiographer, a cassette, or a reading apparatus having abnormalities frequently generated is statistically extracted, the causes of abnormalities are exactly specified, and the removal of the causes can be quickly achieved.

As explained in the foregoing, according to the medical image pickup system 1100 of this embodiment, as the preparation of an image pickup process, image pickup order information for carrying out the image pickup process is transmitted from the control apparatus 120 to the portable terminal 110 and is stored therein, and at the start of the image pickup process, the patient ID of a patient to be subjected to the image pickup processing, the radiographer ID of a radiographer to carry out the image pickup process, the cassette ID of a cassette for recording a medical image, and the image pickup ID of an image pickup apparatus for carrying out the image pickup process are read, and brought in correspondence with the image pickup order information. Further, after the completion of the image pickup process, the radiographer ID, cassette ID, and image pickup apparatus ID are transmitted to the control apparatus 120 in correspondence with the image pickup order information from the portable terminal 110. On the other hand, in the medical image reading apparatus 130, the medical image and the cassette ID are read from the cassette 150 having the medical image recorded, and the medical image are transmitted to the control apparatus 120 with the cassette ID and reading apparatus ID attached. Further, in the control apparatus 120, the radiographer ID, cassette ID, image pickup apparatus ID, and reading apparatus ID are stored in the image pickup history administration file 1261 in correspondence with the image pickup order information, while the image pickup order information and the medical image are brought in correspondence with each other on the basis of the cassette ID and controlled.

By this, it is possible to make definite the radiographer, cassette, image pickup apparatus, reading apparatus involved in the image pickup process of the medical image; therefore, in cases where an abnormality is generated in a medical image, the cause of the abnormality can be quickly specified, and the cause can be removed. Further, because the above-mentioned radiographer ID, image pickup apparatus ID, and reading apparatus ID are attached to the medical image as attached bits of information, and are controlled in correspondence with the image pickup order information, the correspondence relation between each of the bits of identification information and the medical image is made definite, which makes the control easy.

Further, the image pickup history administration file 1261, in the case where an abnormality is generated in a medical image and "NG" is inputted by a radiographer, sets the error flag of the corresponding medical image to ON, and stores the generation of error as error information. By this, for example, on the basis of the image pickup history administration file 1261, the identification information of the radiographer, cassette, image pickup apparatus, and reading apparatus involved in the image pickup process of the medical image having the abnormality generated can be displayed; therefore, the cause of the abnormality being generated can be statistically investigated.

In addition, the description of this embodiment described in the foregoing is an example of a suitable medical image pickup system and a method of controlling medical images of this invention, and this invention should not be limited to this.

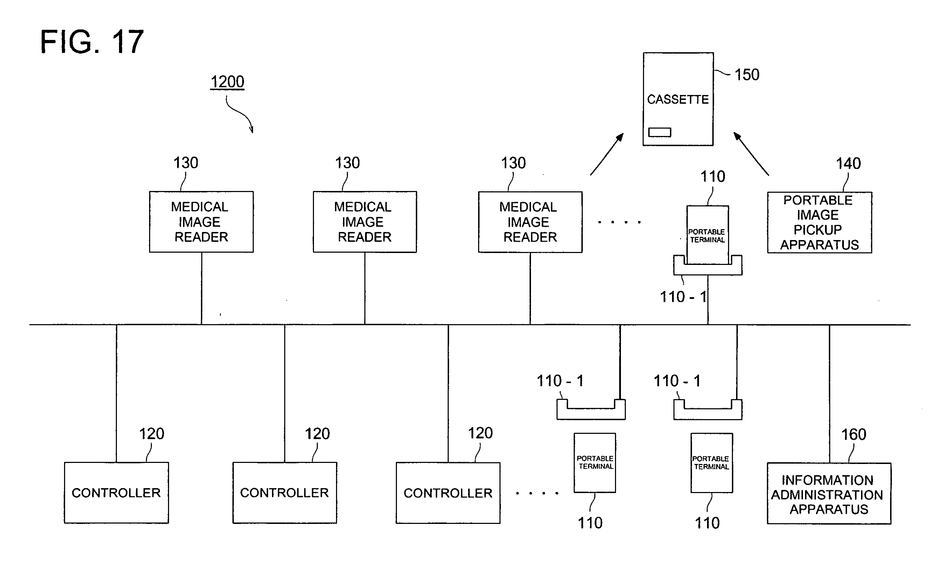

For example, the system structure of the above-mentioned medical image pickup system 1100 is only an example, and this invention is not to be limited to this. It is also appropriate, as a medical image pickup system 1200 shown in FIG. 17, to have a structure such that the portable terminal 110 and the communication terminal 110-1 for controlling the communication with the control apparatus 120 are directly connected with the control apparatus 120, or to have a structure such that they are connected to the network N and are capable of arbitrarily communicating with a plurality of control apparatus 120.

In this medical image pickup system 1200, it is desirable that image pickup order information to be transmitted from the control apparatus 120 to the portable terminal 110 includes information on the transmission end of a cassette ID which has been brought in correspondence with the order information of the finished image pickup process (for example, an IP address for the identification of a computer on the network). That is, because information can be arbitrarily transmitted to the plurality of control apparatus 120 connected with the network N, the CPU 111 of the portable terminal 110 having detected the connection with the communication terminal 110-1 acquires the transmission end of the cassette ID from the image pickup order information, and transmits the cassette ID having been brought in correspondence with the image pickup order information to the control apparatus 120 corresponding to the acquired transmission end.

Further, it is also appropriate to have a structure such that the portable terminal 110 stores the identification information of the control apparatus 120 having received image pickup order information, and in the case where the information on the transmission end is not included in the image pickup order information received from the control apparatus 120, transmits the cassette ID having been brought in correspondence with the image pickup order information to the control apparatus 120 having received the image pickup order information concerned. In another case, it is also appropriate to have a structure such that the transmission end is inputted from the input section 112 of the portable terminal 110, and necessary information is transmitted to said designated transmission end.

By this structure, wherever a radiographer is in a hospital, he can transmit information from the portable terminal 110 to the desired control apparatus 120; therefore, for example, in a case where information is required in a hurry, the necessary information can be quickly transmitted. Further, even in a case where the information concerned has a volume possibly exceeding the capacity of the storage device 116 of the portable terminal 110, it is possible to quickly transmit the information to achieve the backup of the data without going to the image pickup room where the control apparatus 120 is disposed; this makes the convenience of the system good.

Further, the explanation has been made in such a manner that the system has a structure such that a radiographer ID, cassette ID, image pickup apparatus ID, etc. are acquired by means of the bar code 117 of the portable terminal 110; however, this invention is not limited to this, and also it is appropriate to make the structure such one that the ID card of a radiographer, the cassette 150, and the portable image pickup apparatus 140 each are equipped with an IC chip having the identification information recorded, and the identification information recorded in the IC chip is acquired by the portable terminal 110. In this case, the portable terminal is made to be equipped with an IC reader (not shown in the drawing).

Further, the information to be stored in the image pickup history administration file 1261 is not limited to the above-mentioned examples, but various kinds of information can be stored. For example, it is also appropriate to have a structure such that image pickup order information is recorded in an image pickup history administration file, or it is also appropriate to have a structure such that, as information on the things relating to an image pickup process, the identification number of the portable terminal 110, the identification number of the communication terminal 110-1, the image processing condition at the time of image processing, the ID of a radiographer having practiced the image processing, etc. are stored in correspondence with the image pickup order information. Further, it is also appropriate to have a structure such that an operation to make correspondence with image pickup order information is carried out on the basis of various kinds of information such as an image pickup ID, cassette ID, and patient ID. Further, as regards the correspondence relation between image pickup information and a medical image, it is enough so long as the correspondence relation between them is definite in the same way on the basis of various kinds of information.

Further, the time a radiographer ID, cassette ID, and image pickup apparatus ID are acquired is not to be limited to the above-mentioned example, but it is possible to acquire those ID's at an arbitrary time so long as the correspondence relation with the image pickup order information is definite. For example, it is also appropriate to have a structure such that the radiographer ID is included in the image pickup order information transmitted from the control apparatus 120 to the portable terminal 110 in an image pickup preparation processing; in this case, it is also appropriate that the portable terminal 110 carries out only the certification of the radiographer ID in the image pickup start processing. In another case, it is also appropriate in the case of post-image pickup register where the cassette ID is registered after the completion of an image pickup process, to make the cassette ID not be acquired before the start of the image pickup process.

In addition to the above-mentioned, as regards the detailed structure and detailed operation of the medical image pickup apparatus 100 and 200 in the embodiment of this invention, it is a matter of course that they can be suitably changed within the scope not departing from the intention of this invention.

According to the embodiment 2, in correspondence with image pickup order information, identification information of a radiographer having carried out the image pickup process, identification information of a cassette having a medical image recorded, and identification information of an image pickup apparatus having recorded a medical image are stored; therefore, it is possible to identify the radiographer, the cassette, and the image pickup apparatus having been involved in the image pickup process speedily. By this, in cases where an abnormality is generated in a medical image, it is possible to specify whether or not the cause does exist in any one of the technician, cassette, and image pickup apparatus, which makes it possible to quickly remove the cause of the abnormality generation.

According to the embodiment 2 of this invention, further, it is possible that the radiographer, cassette, image pickup apparatus, and reading apparatus involved in the image pickup process are stored in the image pickup history administration file and controlled in correspondence with the image pickup order information, while the medical image is controlled in correspondence with the image pickup order information. Owing to this, for example, in cases where an abnormality is generated in a medical image, on the basis of the image pickup history administration file, the cause of the abnormality can be searched through the inspection of various factors to becomes the cause such as the radiographer, cassette, image pickup apparatus, and reading apparatus involved in the image pickup process, and the cause of the generation of abnormality can be removed with certainty.

According to the embodiment 2 of this invention, furthermore, it is possible that a medical image is made to have identification information of a cassette, identification information of a radiographer, identification information of an image pickup apparatus, and identification information of a reading apparatus attached as attached bits of information and the medical image is controlled in correspondence with image pickup order information; therefore, the correspondence relation between a medical image and various kinds of identification information can be made definite, and the loss of information can be prevented.

According to the embodiment 2 of this invention, furthermore, by the storing of an abnormality being present for medical images having ever had an abnormality generated, the causes of abnormality can be extracted statistically on the basis of medical images having ever had an abnormality generated; therefore, the cause of abnormality generated in a medical image can be searched easily with a high efficiency.

## Claims

1. A medical image pickup system, comprising:
a plurality of cassettes, each of which comprises a recording medium and identification information of each cassette;
a plurality of reading apparatuses for reading out a medical image from a cassette of the plurality of cassettes, each of the plurality of reading apparatuses having identification information of each of the plurality of reading apparatuses;
a control apparatus for correlating an image pickup order information and the medical image using the identification information of a cassette,
the image pick up system further comprising:
an image pickup history generation section for correlating the read out image with the identification information of the cassette and the identification information of the reading apparatus which are used for reading the image among the plurality of reading apparatuses; and
a storing section for storing a correlation information of the image, the identification information of the cassette, and. the identification information of the reading apparatus.

2. The medical image pickup system according to claim 1, further comprising a plurality of control apparatuses, each of which has an image processing section and identification information of each control apparatus, wherein when each of the plurality of control apparatuses performs an image processing, the storing section stores the identification information of the control section correlating with a processed image.

3. The medical image pickup system according to claim 2, wherein at least one of the plurality of control apparatuses comprises the image pickup history generation section.

4. The medical image pickup system according to claim 1, further comprising:
a portable terminal for acquiring image pickup order information from the control apparatus; wherein the portable terminal comprises:
an acquisition device for acquiring at least one of identification information of a cassette having a medical image recorded, identification information of a radiographer having carried out an image pickup operation, and identification information of an image pickup apparatus having recorded a medical image into the cassette;
a storage device for storing the acquired identification information in correspondence with the image pickup order information; and
a first transmission device for transmitting the identification information in correspondence with the image pickup order information;
wherein the control apparatus comprises:
a communication device for receiving the identification information correlated with the image pickup order information; and
an image pickup history administration file for storing the received identification information correlating with the image pickup order information.

5. The medical image pickup system according to claim 4, wherein the reading apparatus comprises:
a second transmission device for transmitting identification information of the cassette and the identification information of the reading apparatus having read the medical image, in correspondence with the medical image;
wherein the communication device in the controlling apparatus receives the medical image brought in correspondence with the identification information of the cassette and the identification information of the reading apparatus, and
the image pickup history administration file stores the identification information of the reading apparatus in correspondence with the identification information including the identification information of the cassette,
wherein the medical image pickup system further comprises an administration device for correlating and controlling the medical image and the image pickup order information on the basis of the identification information of the cassette.

6. The medical image pickup system according to claim 5, wherein the administration device attaches to the medical image at least one of the identification information of the cassette, the identification information of the radiographer, the identification information of the image pickup apparatus, and the identification information of the reading apparatus as image-attached information.

7. The medical image pickup system according to any one of claims 4 to 6, wherein the control apparatus further comprises:
a display control device for making a display section display the medical image; and
an input control device for inputting error information indicating whether or not an abnormality is present in the medical image displayed,
wherein the image pickup history administration file stores error information in correspondence with the identification information corresponding to the medical image.

8. A method of administrating medical images in a medical image pickup system equipped with a control apparatus for correlating and controlling image pickup order information and a medical image, and a portable terminal for acquiring image pickup order information from the control apparatus, the method of administrating medical images comprising;
acquiring at least one of identification information of a cassette having a medical image recorded, identification information of a radiographer having carried out the image pickup, and identification information of an image pickup apparatus having recorded a medical image in the cassette;
storing the acquired identification information in correspondence with the image pickup order information;
transmitting the identification information in correspondence with the image pickup order information;
receiving the identification information brought in correspondence with the image pickup order information; and
storing the received identification information in correspondence with the image pickup order information concerned.

9. The method of administrating medical images according to claim 8, further comprising:
reading, from a cassette having a medical image recorded, the medical image and the identification information of the cassette;
transmitting the identification information of the cassette and the identification information of the reading apparatus having read the medical image, in correspondence with the medical image;
receiving the medical image having the identification information of the cassette and the identification information of the reading apparatus attached in correspondence;
storing the identification information of the reading apparatus in correspondence with various kinds of identification information including the identification information of the cassette; and
administrating the medical image and image pickup order information in correspondence with each other on the basis of the identification information of the cassette.

10. The method of administrating medical images according to claim 9, further comprising a step of attaching to the medical image at least one of the identification information of the cassette, the identification information of the radiographer, the identification information of the image pickup apparatus, and the identification information of the reading apparatus.

11. The method of administrating medical images according to any one of claims 8 to 10, further comprising:
displaying the medical image on a display;
inputting error information indicating whether or not an abnormality is present in the medical image displayed; and
storing error information in correspondence with each of the identification information corresponding to the medical image.
